(19) Europäisches Patentamt
European Patent Office
Office européen des brevets

(11) EP 1 599 258 B1

(12) **EUROPEAN PATENT SPECIFICATION**

(45) Date of publication and mention of the grant of the patent:
**06.08.2008 Bulletin 2008/32**

(21) Application number: **04715148.5**

(22) Date of filing: **26.02.2004**

(51) Int Cl.:
***A61P 7/00*** *(2006.01)* ***A61P 7/04*** *(2006.01)*
***A61P 35/00*** *(2006.01)*

(86) International application number:
**PCT/US2004/005994**

(87) International publication number:
**WO 2004/075989 (10.09.2004 Gazette 2004/37)**

(54) **FUMED SILICA EMBOLIC COMPOSITIONS**

EMBOLIEZUSAMMENSETZUNGEN ENTHALTEND PYROGENES SILICIUM

COMPOSITIONS D'EMBOLISATION DE FUMEE DE SILICE

(84) Designated Contracting States:
**AT BE BG CH CY CZ DE DK EE ES FI FR GB GR HU IE IT LI LU MC NL PT RO SE SI SK TR**

(30) Priority:
**26.02.2003 US 450626 P**
**26.02.2003 US 450289 P**
**26.02.2003 US 450288 P**
**27.02.2003 US 451228 P**
**27.02.2003 US 451310 P**
**07.04.2003 US 461289 P**
**07.04.2003 US 461283 P**
**07.04.2003 US 461290 P**
**07.04.2003 US 461177 P**
**07.04.2003 US 461288 P**

(43) Date of publication of application:
**30.11.2005 Bulletin 2005/48**

(73) Proprietor: **Micro Therapeutics, Inc.**
**Irvine CA 92618 (US)**

(72) Inventors:
- **PATTERSON, William Robert**
  **Irvine, CA 92614 (US)**
- **SLEE, Earl**
  **Laguna Niguel, CA 92677-4243 (US)**
- **WHALEN II, Thomas J.**
  **Encinitas, California 92024 (US)**
- **GREFF, Richard J.**
  **St. Petersburg Beach, Florida 33706 (US)**
- **ROSEN, Meyer R.**
  **East Norwich, New York 11732 (US)**

(74) Representative: **Wibbelmann, Jobst et al**
**Wuesthoff & Wuesthoff**
**Patent- und Rechtsanwälte**
**Schweigerstrasse 2**
**81541 München (DE)**

(56) References cited:
**US-A- 5 273 728**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Cross-Reference to Related Applications

This application claims the benefit under 35 USC § 119(e) of United States Provisional Application Serial No. 60/450,289, filed February 26, 2003; No. 60/461,177, filed April 7, 2003; No. 60/451,310, filed February 27, 2003; 60/461,290, filed April 7, 2003; No. 60/450,626, filed February 26, 2003; No. 60/461,289, filed April 7, 2003; No. 60/450,288, filed February 26, 2003; No. 60/451,228, filed February 27, 2003; No. 60/461,288, filed April 7, 2003; No. 60/461,283, filed April 7, 2003 which are hereby incorporated by reference in their entirety.

### Field of the Invention

This invention is directed to fumed silica embolic compositions suitable for use in embolizing vascular sites which are in need of embolization due to aneurysms, arteriovenous fistulae, arteriovenous malformations, tumors, and other vascular diseases. They also find use in tissue augmentation settings. This invention is also directed to kits of parts comprising fumed silica comprising embolic compositions suitable for use in embolizing vascular sites which are in need of embolization due to aneurysms, arteriovenous fistulae, arteriovenous malformations, tumors, and other vascular diseases. In addition, devices for delivering these compositions to vascular sites are provided.

### References

The following literature and patent publications are cited in this application as superscript numbers.

1 Greff, et al., U.S. Patent No. 5,851,508, "Compositions for Use in Embolizing Blood Vessels" issued December 22, 1998.

2 Whalen II, et al., U.S. Patent No. 6,645,167, "Methods for Embolizing Vascular Sites with an Embolic Composition" issued November 11; 2003.

3 Whalen, II, et al., U.S. Patent No. 6,531,111, "High Viscosity Embolizing Compositions" issued March 11, 2003.

4 S.C. Porter, U.S. Patent Application Publication No. 20030039696, "Embolic Compositions with Non-cyanoacrylate Rheology Modifying Agents" published February 27,2003.

5 C. Porter, et al., U.S. Patent Application No. 10/686,929, "Polymeric Materials for Site Specific Delivery to the Body" filed October 15, 2003.

6 C. Porter, et al., U.S. Patent Application No. 10/687,545, "Prepolymeric Materials for Site Specific Delivery to the Body" filed October 15, 2003.

7 Evans, et al., U.S. Patent No. 5,695,480, "Embolizing Compositions" issued December 9, 1997.

8 Greff, U.S. Patent Application Serial No. 10/162,653, "Novel High Viscosity Embolic Compositions Comprising Prepolymers" filed June 6, 2002.

9 Greff, et al., U.S. Patent No. 6,248,800, "Methods for Sterilizing Cyanoacrylate Compositions" issued June 19, 2001.

10 Hademmenos & Massoud, (1998), The Physics of Cerebrovascular Diseases, Springer-Verlag, New York, USA.

11 Bird, Stewart, & Lightfoot (1960), Transport Phenomena, John Wiley & Sons, New York, USA.

12 Braun & Rosen (2000), Rheology Modifiers Handbook: Practical Use and Application. William Andrew Publishing, New York, USA.

13 Cabot Corp (2000), Cab-O-SIL Untreated Fumed Silica: Properties and Functions, Cabot Corp, Illinois, USA.

14 Porter, U.S. Patent Application Publication No. 20020165582, "Method and Apparatus for Delivering Materials to the Body" published November 7, 2002.

15 Greff, et al., U.S. Patent No. 5,667,767 for "Compositions for Use in Embolizing Blood Vessels" issued September 16, 1997.

16 Whalen, et al., U.S. Patent No. 6,645,167 for "Methods for Embolizing Aneurysmal Sites With a High Viscosity Embolizing Composition" issued November 11, 2003.

17 Mandai, et al., "Direct Thrombosis of Aneurysms with Cellulose Acetate Polymer", J. Neurosurg., 77:497-500 (1992).

18 Kinugasa, et al., "Direct Thrombosis of Aneurysms with Cellulose Acetate Polymer", J. Neurosurg., 77:501-507 (1992).

19 Greff, et al., U.S. Patent No. 5,580,568 for "Cellulose Diacetate Compositions for Use in Embolizing Blood Vessels" issued December 3, 1996.

20 Casarett and Doull's Toxicology, Amdur et al., Editors, Pergamon Press, New York, pp. 661-664 (1975).

21 Kinugasa, et al., "Early Treatment of Subarachnoid Hemorrhage After Preventing Rerupture of an Aneurysm", J. Neurosurg., 83:34-41 (1995).

22 Kinugasa, et al., "Prophylactic Thrombosis to Prevent New Bleeding and to Delay Aneurysm Surgery", Neurosurg., 36:661 (1995).

23 Taki, et al., "Selection and Combination of Various Endovascular Techniques in the Treatment of Giant Aneurysms", J. Neurosurg., 77:37-42 (1992).

24 Dunn, et al., U.S. Patent No. 4,938,763 for "Biodegradable In-Situ Forming Implants and Methods of Producing Same", issued July 3, 1990.

## BACKGROUND OF THE INVENTION

Embolization of blood vessels is conducted for a variety of purposes, including the treatment of aneurysms, arteriovenous fistulae ("AVFs"), arteriovenous malformations ("AVMs"), tumors, uncontrolled bleeding, and the like.

Embolization of blood vessels is preferably accomplished via catheter techniques, which permit the selective placement of the catheter at the vascular site to be embolized. Advancements in catheter technology and in angiography now permit neuroendovascular intervention, including the treatment of otherwise inoperable conditions. Advancements in microcatheters and guide wires provide access to vessels as small as 1 mm in diameter, and allow for the endovascular treatment of many lesions.

Embolic compositions heretofore disclosed in the art include those comprising a biocompatible polymer, a biocompatible solvent, and a contrast agent which allow visualization of the *in vivo* delivery of the composition via fluoroscopy. Typically, conventional low-viscosity embolic compositions comprise no more than about 9 weight percent of biocompatible polymer.[7, 15, 17-19, 21-24]

Endovascular treatment regimens preferably include the use of a water-insoluble, radiopaque contrast agent in the embolic compositions in order that the physician can visualize delivery of the composition to the vascular site via conventional techniques such as fluoroscopy. Additionally, the use of water-insoluble contrast agents is beneficial during post-treatment procedures to visualize the embolized mass, for example, to monitor the disease condition and/or retreatment purposes.

Visualization is particularly necessary when using catheter delivery techniques in order to ensure both that the composition is being delivered to the intended vascular site and that the requisite amount of composition is delivered. The latter requirement is particularly critical in the treatment of aneurysms, where only the aneurysmal sac is intended to be filled while leaving the adjoining blood vessel unaffected.

Accordingly, in such treatments, the amount of embolic composition delivered is selected to substantially fill, but not overflow, the aneurysmal sac. If less than this amount of embolic composition is delivered to the aneurysmal sac, the patient will be left with an active aneurysm which, in some cases, may grow/enlarge over time. If more than this amount of embolic composition is delivered, the composition will overflow into the adjoining blood vessel where it then embolize this blood vessel as well as the aneurysm. In the case where the affected blood vessel is in or leads to a critical body organ (*e.g.*, the brain), damage due to blood flow reduction or cessation can result in severe patient disability or death.

When delivered by catheter, the embolic compositions preferably comprise a biocompatible solvent, a biocompatible polymer, and a biocompatible contrast agent. The biocompatible solvent is miscible or soluble in blood or other body fluid and solubilizes the biocompatible polymer during delivery. The biocompatible polymer is selected to be soluble in the biocompatible solvent, but insoluble in blood or other body fluid. The biocompatible contrast agent is suspended in the composition and, as above, permits the physician to fluoroscopically visualize delivery of the composition. Upon contact with the blood or other body fluid, the biocompatible solvent dissipates from the embolic composition, whereupon the biocompatible polymer precipitates in the presence of the biocompatible water-insoluble contrast agent and embolizes the vascular site.

Usually, the embolic compositions used heretofore have had low viscosities to enable them to be easily fed through small diameter catheters. Notwithstanding the benefits associated with the use of such conventional low-viscosity embolic compositions in treating vascular disorders, *in vivo* these compositions often formed masses at points distal from the point of ejection from the catheter. That is, upon ejection of the embolic composition at a vascular site, the coherent mass subsequently formed was often distal, and not proximal, the ejection port of the catheter. Moreover, upon solidification, the solid mass formed was often linear in shape (*i.e.*, having a "string shape").

In many circumstances, a contiguous or ball-shaped precipitate formed at the ejection port is desired (*e.g.*, to fill an aneurysmal sac). Distal solidification of a string-shaped precipitate makes site-specific delivery of the solid mass in the vasculature difficult. As is apparent, site-specific delivery of the solid mass is essential for treatment of vascular disorders such as aneurysms. Solidification at points distal to the ejection port, as is common in string-shaped precipitates, can result in a solid mass forming not in the aneurysmal sac, but in an artery attendant the aneurysm. Such a string-shaped precipitate is more prone to fragmentation, which can lead to embolization of the attendant artery and to possible incapacitation or death of the patient. Moreover, fragmentation can lead to particles or fragments being "washed" downstream and lodging at undesired locations in the vasculature.

In addition, the lower viscosity embolizing compositions of the art are prone to leakage when being delivered to aneurysms. As noted, this leakage is especially pronounced prior to completion of aneurysm fill. In other procedures, such as treatment of AVM's, tumors and the like, lower viscosity compositions are desirable. Nonetheless, even in these settings higher viscosities can lead to desirable reduction in leakage.

To address these problems, higher viscosity embolic compositions were developed which provided for contiguous or ball-shaped precipitates and a reduced frequency of unintended embolization such as leakage in parent artery when treating an aneurysm.[15,16] The high viscosity of these compositions, however, often required the use of reinforced high-pressure microcatheters and the like.

In view of the foregoing, it is apparent that there is an ongoing need to improve embolic compositions such that they are biocompatible, easily administered, and effective at providing controlled site-specific embolization.

## SUMMARY OF THE INVENTION

This invention is directed to novel compositions suitable for use as embolic compositions comprised of fumed silica, use of such compositions for embolizing vascular sites, kits of parts comprising such compositions and improved composition delivery devices which administer these compositions. The invention is premised on the discovery that facile in vivo delivery of an increased viscosity composition may be achieved by using fumed silica as a rheological modifier in the composition. Surprisingly, these modified biocompatible compositions flow in the delivery catheter with acceptable viscosity difficult to attain with high viscosity materials, but embolize vascular sites better than a low viscosity composition. The higher viscosity at rest, coupled with lower viscosity under shear can be optimized by adjusting composition parameters to achieve substantially reduced leakage from the site of use while attaining reasonable processes and other process techniques during delivery.

Without being limited to any theory, it is believed that addition of fumed silica to a composition promotes pseudoplastic behavior in the composition. At rest, the composition has a high viscosity or yield stress. However, when stress is applied to the composition, such as the stress applied to it as it moves through a delivery catheter, low energy bonds (*e.g.*, hydrogen bonds or van der Waals bonds) are broken in the composition, and the viscosity under higher shear rates is significantly lowered.

The compositions of the invention comprise: (1) a biocompatible polymer that is insoluble in blood or other body fluids; (2) a biocompatible solvent that is miscible in blood and other body fluids and serves to solubilize the biocompatible polymer; (3) a biocompatible contrast agent that is suspended in the composition; and (4) a sufficient amount of fumed silica to act as a rheological modifier in the composition and to impart a shear thinning index to the composition (as that parameter is determined at 1.0 $s^{-1}$ and 10.0 $s^{-1}$ as set forth herein) of at least 4 and preferably of from 4.5 to 6.5.

This invention can be applied advantageously to relatively viscous compositions used to embolize aneurysms as well as the lower viscosity composition more typically employed in the treatment of an AVM and the like. Intermediate viscosity composition can serve both needs.

Representative high viscosity embodiments achieved using this invention have a viscosity of at least 25,000

and especially at least 50,000 cP at 0.24 $s^{-1}$ and 37°C and a viscosity no greater than 5,000 cP under shear when a shear rate of at least 100 $s^{-1}$ at 37°C is applied. The viscosity of representative intermediate viscosity compositions is at least 4000 cP and 0.24 $s^{-1}$ and 37°C and no greater than 2000 cP at 37°C when a shear rate of at least 100 $s^{-1}$ is applied. Preferably, these compositions are used to embolize a vascular site for the purpose of treating one or more of the following conditions; an aneurysm, arteriovenous fistulae, uncontrolled bleeding and the like.

The viscosity of representative low viscosity composition is at least 2000 cP at 37°C and 0.24 $s^{-1}$ and no greater than 500 cP at 37°C when a shear rate of at least 100 $s^{-1}$ is applied. Preferably, these compositions are used to embolize a vascular site for the purpose of treating one or more of the following conditions; arteriovenous malformations, tumors, uncontrolled bleeding and the like.

In another embodiment of the invention, a use for embolizing a vascular site in a mammal is provided, by delivering via a catheter into the vascular site a composition comprising (1) a biocompatible polymer that is insoluble in blood or other body fluids, (2) a biocompatible solvent that is miscible in blood and other body fluids and serves to solubilize the biocompatible polymer, (3) a biocompatible contrast agent that is suspended in the composition, and (4) to act as a rheological modifier in the composition and to impart a shear thinning index to the composition of at least 4 and preferably of from 4.5 to 6.5. Upon delivery of the composition into the vascular site, the composition decelerates and its viscosity increases while forming a precipitate which embolizes the vascular site. One composition has a viscosity of at least 25,000 and especially at least 50,000 cP at 0.24 $s^{-1}$ and 37°C and a viscosity no greater than 5,000 cP under shear when a shear rate of at least 100 $s^{-1}$ at 37°C is applied. The viscosity of the intermediate viscosity composition is at least 4000 cP at 37°C and 0.24 $s^{-1}$ and no greater than . 2000 cP at 37°C when a shear rate of at least 100 $s^{-1}$ is applied. Preferably, the vascular site in need of embolization is due to one of the following conditions; aneurysm, an arteriovenous fistulae, uncontrolled bleeding and the like.

A preferred composition which meets the viscosity requirement set forth in the higher viscosity composition and in the method just described has a range of biocompatible polymer (weight/volume solvent) to fumed silica (weight/final weight) of from 2.6 to 1 to 3.6 to 1. More preferably, the ratio is from 3.0 to 1 to 3.2 to 1. Even more preferably, the ratio is 3.1 to 1. Such compositions can also be described as having a weight ratio of biocompatible polymer to silica based on the final weight of the composition of from 1.4 to 1 to 0.9 to 1.

The low-viscosity composition of the invention can be used for embolizing a vascular site in a mammal, by delivering via a catheter into the vascular site a composition comprising (1) a biocompatible polymer that is insoluble in blood or other body fluids, (2) a biocompatible solvent that is miscible in blood and other body fluids and serves to solubilize the biocompatible polymer, (3) a biocompatible contrast agent that is suspended in the composition, and (4) a sufficient amount of fumed silica to act as a rheological modifier in the composition. Upon delivery of the composition into the vascular site, the composition decelerates and its viscosity increases while forming a precipitate which embolizes the vascular site. The viscosity of the composition is at least 2000 cP at 37°C and 0.24 $s^{-1}$ and no greater than 500 cP at 37°C when a shear rate of at least 100 $s^{-1}$ is applied. Preferably, the vascular site in need of embolization is due to one of the following conditions; arteriovenous malformation, tumor, uncontrolled bleeding and the like.

In other embodiments, kits of parts are provided that comprise a composition as described above and a catheter and/or a syringe selected for access and embolization associated with the specific condition being treated.

In addition, it is contemplated that any of these compositions, and especially the less viscous embodiments, could be employed in tissue bulking, and tissue augmentation procedures.

In additional aspects, this invention provides an improved syringe-based system for loading and dispensing these and other similar relatively viscous compositions to the vascular points of use.

## Brief Descriptions of the Drawings

FIG. 1 illustrates the change in non-Newtonian viscosity versus shear rate relationship for an embolic composition comprising amorphous, hydrophilic, fumed silica as a rheological modifier both before and after sterilization.

FIG. 2 illustrates the presence of surface hydroxyl groups in the silanol portion of amorphous, hydrophilic, fumed silica.

FIG. 3A illustrates the hydrogen bonding between two surface hydroxyl groups of the silanol portion of amorphous, hydrophilic, fumed silica whereas FIG. 3B illustrates the network of silica particles arising under static conditions due to the hydrogen bonding illustrated in FIG. 3A.

FIG. 4 illustrates a mixing chamber for use in mixing an embolic composition comprising a hydroxyl-containing silica rheological modifier and the vortices formed during mixing.

FIG. 5 illustrates the non-Newtonian viscosity versus shear rate relationship of four different embolic compositions comprising amorphous, hydrophilic, fumed silica over a variety of shear rates.

FIG. 6 illustrates the main effects plot showing contributing curves of the input variables (weight/final weight) for control at the neck of the aneurysm. The horizontal line represents the highest average score generated for the neck control response variable.

FIG. 7A illustrates the properties of a composition identified as Formula K, as well as other composition in a Casson plot.

FIG. 7B illustrates Formula K and related compositions in a Power Law plot.

FIG. 8A illustrates a cross-section of a canine carotid artery which was embolized using an embolic composition not containing fumed silica.

FIG. 8B illustrates a cross-section of a canine carotid artery which was embolized using an embolic composition of this invention.

FIG. 9 illustrates in schematic partially exploded side view a device for delivering the rheologically-modified compositions. In this view the parts involved in filling a special vented-barrel syringe with composition provided in an aluminum squeeze tube are shown.

FIG. 10 illustrates in schematic partially exploded side view the device depicted in FIG. 10 now configured to accurately and controllably administer the rheologically-modified composition to a catheter for delivery to a point of use.

FIG. 11 illustrates in a side view a puncture cap and syringe interface which is part of the syringe filling set up depicted in FIG. 9.

FIG. 12 illustrates in a cross-sectional side view the interface depicted in FIG. 11.

FIG. 13 illustrates in a side view the vented barrel syringe body which makes up part of the systems shown in FIG. 9 and FIG. 10.

FIG. 14 illustrates in distal-end view the syringe body showing its generally oblong handle.

FIG. 15 through FIG. 25 all illustrate various aspects of a "Quick Stop" mechanism which permits the immediate coupling and decoupling of a lead screw drive mechanism for accurately, manually controlling the delivery of the rheologically-modified compositions from a syringe barrel.

More particularly FIG. 15 illustrates a generally top and side perspective view of the Quick Stop mechanism.

FIG. 16 illustrates a generally bottom and end perspective view of the Quick Stop mechanism.

FIG. 17 illustrates in side cross-sectional view the Quick Stop mechanism shown engaging the oblong handle or flange of the syringe barrel illustrated in FIG. 13 and FIG. 14.

FIG. 18 illustrates in bottom perspective view the Quick Stop mechanism and shows how the parts of the mechanism move relative to one another to engage and disengage the lead screw.

FIG. 19 illustrates a bottom view of the Quick Stop mechanism.

FIG. 20 illustrates a bottom view of the base of the Quick Stop mechanism.

FIG. 21 illustrates an end view of the base of the Quick Stop mechanism.

FIG. 22 illustrates a cross-sectional side view of the base of the Quick Stop mechanism.

FIG. 23 illustrates a bottom view of the activator of the Quick Stop mechanism.

FIG. 24 illustrates a side view of the activator.

FIG. 25 illustrates in top view, the threaded pincher which grips and releases the threaded syringe plunger to effect the Quick Stop function.

## Detailed Description of the Invention

### Definitions And Overview

As discussed above, the present invention is directed to novel embolic compositions containing fumed silica as a rheological modifier. The composition of the invention is also suitable in use for embolizing vascular sites. The composition of the invention is particularly well suited for a vascular site, which is in need of embolization due to aneurysms.

It must be noted that as used herein and in the claims, the singular forms "a," "and" and "the" include plural referents unless the context clearly dictates otherwise. Thus, for example, reference to "a contrast agent" in a composition includes two or more contrast agents, reference to "a biocompatible polymer" includes two or more polymers, and so forth. In this specification and in the claims which follow, reference will be made to a number of terms which shall be defined to have the following meanings:

**"Viscosity"** is an inherent property of a fluid that exerts a resistance against the movement of the fluid.[10] In the present setting, the rheologically-modified fluids are Non-Newtonian and the observed viscosity is an "apparent viscosity" and is represented by the Greek letter eta, $\eta$. The common unit of viscosity is centipoise (cP) and the International System of Units (SI) unit is the pascal second or Pa*s.

For the purposes of this application and the above definitions, all viscosity values are determined by a cone/plate instrument sold by Brookfield Engineering (Middleboro, Massachusetts, USA) as model R/S-CPS Rheometer with RS232 interface to Windows®-based PC running Brookfield Rheocalc v2.7 software.

**"Shear rate"** (represented by the Greek letter gamma, $\gamma$, technically, a dot is placed over the letter but will not be done in this application), is proportional to the fluid flow rate. Conceptually, then, the higher the flow rate of a fluid through, e.g., a catheter, the higher the shear rate experienced by the fluid. The unit of shear rate is the reciprocal

second, or 1/s or $s^{-1}$.

**"Shear stress"** (represented by the Greek letter tau, $\tau$) of a fluid is the force per unit area (i.e. pressure) that must be applied to maintain the movement of a fluid. The unit of shear stress is the pascal, or Pa.

Newton's Law of Viscosity relates shear stress to shear rate in the following manner:

$$\tau = \eta \times \gamma$$

**"Yield stress"** is the force per unit area (i.e. pressure) that must be applied to move a fluid from rest. The unit of yield stress is the Pascal, or Pa.

**"Rheology"** is the science of deformation and flow and includes the study of the mechanical properties of gases, liquids, plastics, asphalts, and crystalline materials.[11]

**"Shear Thinning Index"** is the degree of shear thinning over a range of shear rates or rotational speeds. Higher ratios indicate greater shear thinning. Shear thinning index, as used herein, is calculated by dividing the apparent viscosity at 1 $s^{-1}$ by the apparent viscosity at 10 $s^{-1}$.

Embolizing compositions comprising an inorganic fumed silica particulate rheology-modifying agent will exhibit a change in apparent viscosity upon moving from an environment with a first hydrodynamic shear rate to an environment having a second hydrodynamic shear rate. The effect sought is typically referred to as **"shear thinning behavior."** For example, the embolizing composition has a low apparent viscosity when flowing through a microcatheter and a relatively high apparent viscosity when it exits the microcatheter and is no longer flowing.

One particular preferred rheological modifier is amorphous, fumed silica which is a synthetic silicon dioxide ($SiO_2$) that does not have the ordered structure or the health hazards of crystalline silica. Unless otherwise noted in herein, the word "**silica**" denotes amorphous, fumed silicon dioxide. Amorphous, fumed silica is a well-known rheology modifier used in personal care, pharmaceutical, household/industrial, and medical device applications. Typically, fumed silica is an extremely small particle with a large surface area, high purity, and a tendency to have a chain-like morphology. The fumed silica preferably has a BET surface area of 100 $m^2/g$ to 700 $m^2/g$.

Untreated, fumed silica is composed of two chemical groups: siloxane groups (Si-O-Si) and surface silanols (Si-OH) shown as isolated hydroxyls in FIG. 2. [13]

Under static conditions, the silica particles described above form a "string of pearls" network via the isolated hydroxyls from separate particles, which form hydrogen bonds between the particles. Silanol hydrogen bonds (H***O) are shown in FIG. 3A and a model of the silica network is shown in FIG. 3B. [13]

Amorphous, fumed silica containing surface hydroxyl groups as depicted in FIG. 2 and FIG. 3A is sometimes referred to herein as "amorphous, hydrophilic, fumed silica".

When an embolic composition comprising fumed silica is moved *(e.g.,* by syringe-injected flow through a catheter), the silanol network created by the hydrogen bonding between the particles is broken or sheared and the fluid "thins." This change in fluid thickness, or viscosity or yield stress, is known as "shear thinning" and is reversible. That is, if the flowing fluid comes to rest, it will thicken again and repeated application of stress results in a reproducible change in viscosity.

If this reversible shear thinning occurs nearly instantaneously, then the fluid is referred to as **"pseudo-plastic".** If the shear thinning occurs over a period of time, then the fluid is referred to as **"thixotropic".** Thixotropic compositions are evident by measuring the shear stress (Y axis) against shear rate (X axis) at both increasing and decreasing shear rates and determining the extent of the area generated between the two curves. Compositions exhibiting pseudo-plastic behavior have little or no area between these curves whereas thixotropic compositions have measurable areas between thee curves. Specifically, for the purposes of this application, compositions having an area of less than 25,000 Pa/sec between increasing and decreasing shear rates of from 0 to 250 $sec^{-1}$ are deemed to be pseudo-plastic; whereas compositions having an area of greater than 25,000 Pa/sec between increasing and decreasing shear rates of from 0 to 250 $sec^{-1}$ are deemed to be thixotropic. More preferably, this area between the two curves is from 1,000 to 20,000 Pa/sec and, still more preferably, from 1,500 to 15,000 Pa/sec.

The term **"alkylene"** refers to divalent alkyl groups of from 1 to 20 carbon groups, which may be straight chained or branched, and include, for example, ethylene, propylene ($-CH_2CH_2-$, $-CH_2CH_2CH_2-$, $-CH(CH_3)CH_2-$, $-CH_2CH(CH_3)-$) and the like.

The term **"biocompatible contrast agent"** refers to a biocompatible radiopaque material capable of being monitored during injection into a mammalian subject by, for example, radiography. In the methods and compositions of this invention, the contrast agent is preferably water-insoluble (*i.e.*, has a water solubility of less than 0.01 mg/ml at 20°C). Examples of biocompatible water-insoluble contrast agents include tantalum, tantalum oxide, and barium sulfate, each of which is commercially available in the proper form for *in vivo* use. Other biocompatible water-insoluble contrast agents include gold, tungsten, and platinum. Preferred biocompatible water-insoluble contrast agents are those having

an average particle size of 10 μm or less. Water-soluble contrast agents are also suitable for use herein and include, for example, metrizamide. Preferably, the biocompatible contrast agent employed does not cause a substantial adverse inflammatory reaction when employed in *vivo.*

The term **"biocompatible polymer"** refers to polymers which, in the amounts employed, are non-toxic and substantially non-immunogenic when used internally in the patient and which are substantially insoluble in the body fluid of the mammal. The biocompatible polymer can be either biodegradable or, preferably, non-biodegradable.

Biodegradable polymers are disclosed in the art. Examples of biodegradable polymers include, but are not limited to: linear-chain polymers such as polylactides, polyglycolides, polycaprolactones, polyanhydrides, polyamides, polyurethanes, polyesteramides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, poly(malic acid), poly(amino acids), polyvinylpyrrolidone, polyethylene glycol, polyhydroxycellulose, polymethyl methacrylate, chitin, chitosan, and copolymers, terpolymers and combinations thereof. Other biodegradable polymers include, for example, gelatin, collagen, etc.

Suitable non-biodegradable biocompatible polymers include, by way of example, cellulose acetates (including cellulose diacetate), ethylene vinyl alcohol copolymers ("EVOH"), hydrogels (e.g., acrylics), polyacrylonitrile, polyvinylacetate, cellulose acetate butyrate, nitrocellulose, copolymers of urethane/carbonate, copolymers of styrene/maleic acid, and mixtures thereof.

Preferably, the biocompatible polymer employed does not cause a substantial adverse inflammatory reaction when employed in *vivo.* The particular biocompatible polymer employed is selected relative to the viscosity of the resulting polymer solution, the solubility of the biocompatible polymer in the biocompatible solvent, and the like. For example, the selected biocompatible polymer should be soluble in the amounts employed in the selected biocompatible solvent and the resulting composition should have a viscosity suitable for in vivo delivery by the methods of this invention.

The preferred biocompatible polymer is ethylene vinyl alcohol copolymer. Other preferred polymers include cellulose acetate butyrate, cellulose diacetate, polymethyl methacrylate, polyvinyl acetate, copolymers of urethane and acrylates, and the like.

Ethylene vinyl alcohol copolymers comprise residues of both ethylene and vinyl alcohol monomers. Small amounts (*e.g.*, less than 5 mole percent) of additional monomers can be included in the polymer structure or grafted thereon provided such additional monomers do not alter the properties of the composition. Such additional monomers include, by way of example only, maleic anhydride, styrene, propylene, acrylic acid, vinyl acetate and the like.

Ethylene vinyl alcohol copolymers are either commercially available or can be prepared by art-recognized procedures. As is apparent to one skilled in the art, with all other facts being equal, copolymers having a lower molecular weight will impart a lower viscosity to the composition as compared to higher molecular weight copolymers.

As is also apparent, the ratio of ethylene to vinyl alcohol in the copolymer affects the overall hydrophobicity/hydrophilicity of the composition which, in turn, affects the relative water solubility/insolubility of the composition as well as the rate of precipitation of the copolymer in an aqueous environment (*e.g.*, blood or tissue). In a particularly preferred embodiment, the copolymers employed herein comprise a mole percent of ethylene of from 25 to 60 and a mole percent of vinyl alcohol of from 40 to 75. These compositions provide for requisite precipitation rates suitable for use in the methods described therein.

The term **"biocompatible solvent"** refers to an organic material that is liquid at the body temperature of the mammal, in which the composition is used, in which the biocompatible polymer is soluble and, in the amounts used, is substantially non-toxic. Suitable biocompatible solvents include, by way of example, ethyl lactate, dimethylsulfoxide ("DMSO"), analogues/homologues of dimethylsulfoxide, ethanol, acetone, and the like. Aqueous mixtures with the biocompatible solvent can also be employed, provided that the amount of water employed is sufficiently small that the dissolved polymer precipitates upon contact with blood or other bodily fluid. Preferably, the biocompatible solvent is dimethylsulfoxide.

By **"bridging molecule"** is meant a substance which facilitates aggregation of the fumed silica in the composition under static conditions. Suitable bridging molecules include glycols [HO-($C_{2-6}$ alkylene)-OH], wherein alkylene is defined to be straight chained or branched, and derivatives of glycol, such as $[HO-(C_{2-6}\text{ alkylene})-O]_nH$ polymers where n is greater than one and is preferably from 7 to 333.

**"cP"** as used herein refers to a centipoise, which is related to the centistokes by the material's density.

The term **"embolizing"** refers to a process wherein a material is injected into a blood vessel which, in the case of, for example, aneurysms, fills or plugs the aneurysmal sac and/or encourages clot formation so that blood flow into the aneurysm ceases. In the case of AVMs, a plug or clot is formed to control/reroute blood flow to permit proper tissue perfusion. In the case of a vascular site, the vascular site is filled to prevent blood flow therethrough. Embolization of the blood vessel is important in preventing and/or controlling bleeding due to lesions (e. g., organ bleeding, gastrointestinal bleeding, vascular bleeding, and bleeding associated with an aneurysm). In addition, embolization can be used to ablate diseased tissue (e. g., tumors, etc.) by cutting off the diseased tissue's blood supply.

The term **"encapsulation"** as used relative to the contrast agent being encapsulated in the polymer precipitate

does not infer any physical entrapment of the contrast agent within the precipitate, much as a capsule encapsulates a medicament. Rather, this term is used to mean that an integral, coherent precipitate forms which does not separate into individual components.

The term **"rheological modifier"** as used herein refers to a component which when added to the polymer, solvent, and contrast agent composition imparts high rest viscosity or yield stress of the composition (*e.g.*, greater than 2000 cP at 37°C at 0.24 $s^{-1}$ shear rate or a yield stress greater than 20 Pa measured from 37°C shear stress versus shear rate data by the method of Casson) but permits the composition to readily flow under shear stress. Preferably, the rheological modifier is fumed silica. Other hydroxyl containing modifiers could be used.

The term **"rheologically-modified compositions"** refers to compositions comprising the biocompatible polymer, biocompatible solvent, contrast agent and fumed silica as described above.

**"Surfactants"** are those substances which enhance flow and/or aid dispersion by reducing surface tension when dissolved in water or water solutions, or that reduce interfacial tension between two liquids, or between a liquid and a solid. Surfactants also impede the interaction between the rheological modifier and other components of the system. This allows a more fully developed rheological modified system. Surfactants may be anionic, cationic, and nonionic. Surfactants include detergents, wetting agents, and emulsifiers. Suitable cationic surfactants include organic amines and organic ammonium chlorides (e.g., N-tallow trimethylene diamine diolealate and N-alkyl trimethyl ammonium chloride) and the like. Suitable anionic surfactants include, by way of example sulfosuccinates, carboxylic acids, alkyl sulfonates, octoates, oleates, stearates, and the like. Suitable nonionic surfactants, include by way of example, bridging molecules discussed above, Tritons, Tweens, Spans and the like. Polyfunctional additives such as glycerin and various glycols may be added. The adjustment of pH by the addition of potassium or sodium hydroxide ionizes silanols and alters the composition's rheology.

## Compositions

### A. Methods of Making the Rheologically Modified Embolic Compositions

The biocompatible rheologically-modified compositions employed in this invention are prepared by conventional methods, whereby each of the components is added and the resulting composition mixed. For example, these compositions can be prepared by adding sufficient amounts of a biocompatible polymer to a biocompatible solvent which solubilizes said biocompatible polymer. If necessary, gentle heating and stirring can be used to effect dissolution of the biocompatible polymer into the biocompatible solvent (*e.g.*, 12 hours at 50°C). Excessive heating should not be used in order to prevent evaporation of the solvent.

The polymers recited herein are commercially available but can also be prepared by methods known in the art. For example, polymers are typically prepared by conventional techniques such as radical, thermal, UV, $\gamma$ irradiation, or electron beam-induced polymerization employing, as necessary, a polymerization catalyst or initiator to provide for the polymer composition. The specific manner of polymerization is not critical and the polymerization techniques employed do not form a part of this invention.

In order to maintain solubility in the biocompatible solvent, the polymers described herein are preferably not cross-linked.

Sufficient amounts of the contrast agent are then added to the biocompatible solvent to achieve the effective concentration for the composition. In order to enhance formation of a homogenous suspension, the particle size of water-insoluble contrast agents is preferably maintained at 10 $\mu$m or less and more preferably at from 1 to 5 $\mu$m (*e.g.*, an average size of 2 $\mu$m).

After addition of the polymer and contrast agent to the solvent, the fumed silica is added under ambient conditions, preferably under inert atmosphere, for example, an argon atmosphere. The composition is initially stirred at low RPM (less than 1000 RPM) to wet the surface of the silica. Once wetted, the stir rate is increased to a peripheral tip speed of from 5 m/sec to 26.5 m/sec. The tip speed should be maintained until no granular material is evidenced in the composition.

The initial viscosity of the composition is controlled at least in part by the amount of polymer employed and/or its molecular weight. For example, high-viscosity compositions which employ low concentrations of polymer can be achieved by the use of very high molecular weight biocompatible polymers (*e.g.*, those with an average molecular weight greater than 250,000). In the alternative, an high-viscosity composition may be achieved with the use a low molecular weight polymer at a high concentration. Such factors are well known in the art and modification of these parameters will be well within the abilities of one of skill in the art.

The viscosity of the composition is then modified by the addition of the fumed silica. The addition of the silica provides a decrease in the viscosity under shear stress and an increase in the viscosity and/or yield stress under static conditions.

The rheologically-modified composition is stirred as necessary to achieve homogeneity of the composition.

Preferably, mixing/stirring of the composition is conducted under an anhydrous atmosphere at ambient pressure.

In one preferred embodiment the rheologically-modified composition is first mixed in a mixer at a low shear (stir) rate wherein the peripheral tip speed of the mixing blades is less than 10 m/s, and more preferably 1 m/s.

In another preferred embodiment, the rheologically-modified composition is then mixed in a mixer at a high shear (stir) rate wherein the peripheral tip speed of the mixing blade is from 5 m/sec to 26.5 m/sec.

In a particularly preferred embodiment, high shear dispersion (HSD) of the silica in the embolic composition is achieved by a peripheral tip speed (PTS) of from 9 m/sec to 20 m/sec. The PTS measures how much circumferential distance the mixing blade travels per unit time and can be calculated from the revolutions per minute (rpm) and the known dimensions of the mixing blade per the following formula:

$$\text{PTS (m/sec)} = (\text{RPM})(3.14)(\text{Diameter of the Blade in m})(1\ \text{minute}/60\ \text{sec})$$

Preferably, the blade should be 0.5 to 1 blade diameter above the bottom of the mixing vessel. Preferably, the diameter of the mixing vessel should be 2 to 3 times the diameter of the mixing blade.

FIG. 4 illustrates a prototypical mixing blade and mixing vessel. Specifically, mixing vessel 5 comprises a vessel chamber defined by walls 6 and a mixing blade 7 having a blade diameter D. Upon rotation of mixing blade 7, the fluid inside the vessel chamber will be mixed in a manner to define 4 separate vortices denoted by 1, 2, 3 and 4.

If desired, the rheologically-modified composition can be degassed either prior to or after the rheological modifier is added. The degassing can be performed by any conventional degassing technique, *i.e.*, vacuum treatment. In one suitable process, the post-mix material is placed under vacuum for degassing for a prolonged period (*e.g.*, 40 millibar for at least 12 hours).

Optionally, coated rheological modifier, such as coated silica may be employed as a particulate rheological modifier in the rheologically-modified composition. The coating on the rheological modifier is preferably biocompatible and preferably coated in a polymer that is insoluble in both water and biocompatible solvent, e.g. DMSO. The coating is also preferably non-toxic or biocompatible.

In another alternative embodiment, the rheological modifier can be added to the biocompatible solvent, e.g., DMSO, before the mixing of the overall composition. For example, in one embodiment, the solvent could be divided into two portions wherein one portion equaling 1/3 of the total solvent is added to the polymer. Meanwhile, the remaining 2/3 is blended with the rheological modifier. The rheological modifier and the biocompatible solvent is blended to a homogenous composition and then blended with the composition to form the rheologically-modified composition.

The method for manufacturing a rheologically-modified composition as set forth above is only one permutation of the methods for manufacturing the rheologically-modified composition. It can be appreciated that the rheological modifier could be initially blended with the polymer and then added to the solvent and contrast agent, or any combination, including but not limited to adding the rheological modifier to the polymer, solvent or contrast agent or any combination thereof.

Scheme 1 below illustrates protocols for preparing each of the components ultimately employed in the embolic compositions and their use in the methods described above.

SCHEME 1

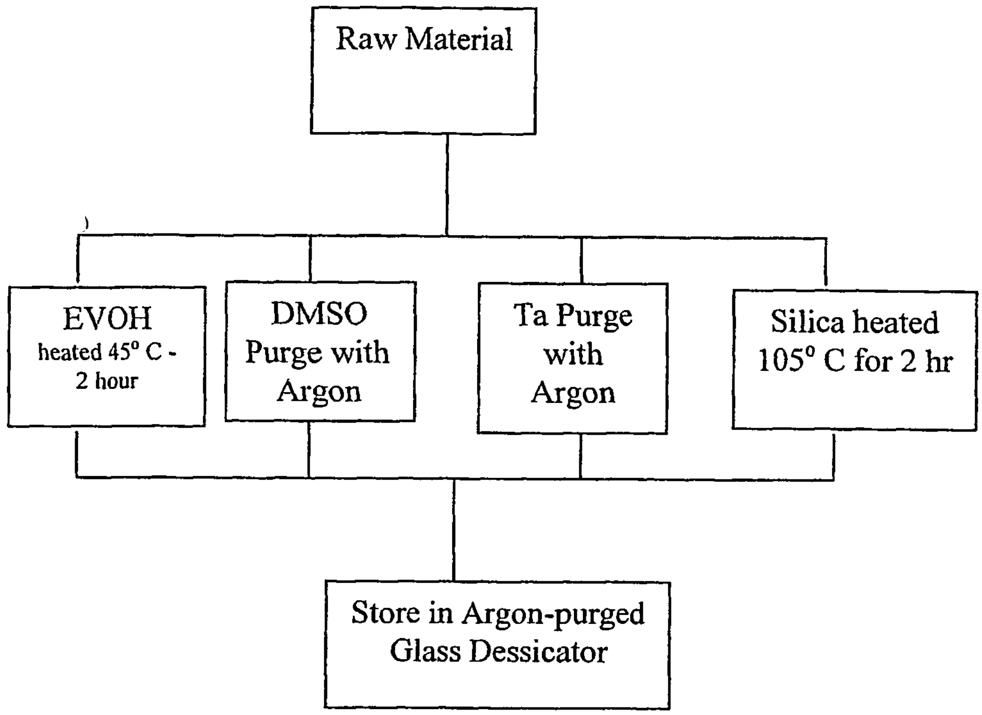


It is understood that these preferred components are merely representative of other components and that other particulate agents other than silica can be treated in a similar manner as silica in Scheme 1. Likewise, biocompatible polymers other than ethylene vinyl alcohol copolymer (EVOH), biocompatible solvents other than DMSO and water insoluble contrast agents other than tantalum (Ta) can be treated in a similar manner to EVOH, DMSO and Ta respectively.

Preferably compositions meeting the above criteria, *i.e.*, higher viscosity, would be used in the treatment of aneurysms, arteriovenous fistulae. When treating such conditions, it is desirable that the composition have proximal occlusion at the vascular site. In other words, it is desirable that the composition act like a plug.

At times percentages are expressed in conventional relative weight percent of the final product and at other times the percentages are expressed as illustrated below.

A preferred composition has a range of biocompatible polymer (weight/volume solvent) to fumed silica (weight/final weight) of from at least 2.6 to 1 to 3.6 to 1. More preferably, the ratio is 3.0 to 1 to 3.2 to 1. Even more preferably, the ratio is about 3.1 to 1.

This ratio is rather unconventional but is an artifact of the way these compositions are assembled. In assembly, a weight in grams of biocompatible polymer is dispersed/dissolved in 100 ml of solvent. A weight of contrast agent is added and a weight addition of fumed silica is incorporated. The ratio of polymer as the initial weight in grams (g) per volume of solvent in milliliters (ml) then divided by the weight of silica in the weight of final product in percent.

For example when 19 g of EVOH are dispersed in 100 ml DMSO (D=1.10, 100 ml = 110 g) and tantalum is added to yield 38% weight to weight of the final product and silica is added to yield 6.175% weight to final weight of the product, the ratio is 19/6.175 or 3.1 to 1.

Alternatively, the amounts of these polymer and modifier materials can be defined by a more conventional ratio of the percent by weight biocompatible polymer to percent weight of fumed silica, both based on the final weight of the composition. This ratio ranges from 1.4 to 1 to 0.9 to 1, especially 1.3 to 1 to 1.0 to 1 and preferably 1.1 to 1.

Based on *in vitro* and *in vivo* testing, four rheologically-modified compositions employing the following ratios of amorphous fumed silica (silica) as the rheological modifier and ethylene vinyl alcohol copolymer (EVOH) as the biocompatible polymer were determined to provide overall beneficial properties both during delivery and upon ejection into aneurysmal sac:

Formula I: 6.25 % silica (wt/final wt) and 7.83 % EVOH (wt/final wt)
Formula J: 6.28 % silica (wt/final wt) and 7.41 % EVOH (wt/final wt)
Formula K: 6.175 % silica (wt/final wt) and 8.21 % EVOH (wt/final wt)

Formula L: 6.38 % silica (wt/final wt) and 6.98 % EVOH (wt/final wt)

These formulae can be generalized as containing from 6.0 to 6.5 % by weight silica and 6.0 to 9.0 % by weight of ethylene vinyl alcohol copolymer.

Each of the above formulae contain approximately 38 % tantalum (wt/final wt) and the remainder of the composition is DMSO.

FIG. 5 illustrates the non-Newtonian viscosities of these compositions over a range of shear rates. Specifically, FIG. 5 illustrates that these compositions exhibit nominal viscosities at high shear rates and rapidly increasing viscosities at low shear rates.

In another preferred embodiment, the rheologically-modified composition has a viscosity under static conditions of at least 2000 cP. It will be appreciated that viscosity at static conditions is derived by extrapolating measurements made at very low shear rates. With modem equipment reproducible values at 0.12 to 0.24 $s^{-1}$ shear rates can be obtained and extrapolated to static values.

Preferably compositions meeting this criteria, *i.e.*, lower viscosity, would be used in the treat of arteriovenous malformations, tumors, and the like. In this instance, it is not undesirable for the composition to have distal occlusion and the lower the viscosity, the easier the delivery.

Particularly preferred rheologically-modified compositions, both in the higher and lower viscosity ranges discussed above, are shown in the table below. All of the % values are % weight based on the weight of the final product:

| **Component** | **Preferred** | **Particularly preferred** |
|---|---|---|
| Biocompatible Polymer | 1 to 12 % | 3 to 9% |
| Contrast Agent | 20 to 55% | 37 to 40 % |
| Fumed Silica | 1 to 12 % | 3 to 10% |

**B. Sterilization**

Sterilization of the embolic compositions prepared as above preferably proceeds via irradiation techniques including, for example, electron beam sterilization, gamma irradiation, and the like. More preferably, compositions of this invention are sterilized via electron beam irradiation.

In another embodiment, the rheologically-modified composition is sterilized by dry heating the composition under conditions sufficient to sterilize the composition. In one embodiment, the rheologically-modified composition is sterilized at 130°C $\pm$ 5°C for approximately 90 minutes.

The table below demonstrates values of thixotropy on sterilized embolic compositions comprising partially treated silica (approximately 50% of the surface silanol groups have been reacted) and completely treated silica as the rheological modifier as compared to a non-sterile embolic composition comprising untreated silica as the rheological modifier.

| **Rheological Modifier / Condition** | Thixotropy (Pa/s) | Increase from non-sterile (fold) |
|---|---|---|
| Untreated silica / Non-sterile | 4,657 | Not Applicable |
| Untreated silica / Heat sterile | 31,058 | 6.669 |
| Partially treated silica / Heat sterile | 10,948 | 2.351 |
| Completely treated silica / Heat sterile | 3,485 | 0.7483 |

Use of partially treated silica as the rheological modifier provides a sterilized embolic composition exhibiting a decreased pseudo-plastic behavior (area = approximately 11,000 Pa/sec) but nevertheless sufficiently pseudo-plastic to be useful in this invention. The use of completely treated silica provides for a sterilized embolic composition exhibiting even better pseudo-plastic behavior as compared to both other compositions.

**C. Other Components**

Surfactants can be optionally employed in the biocompatible rheologically-modified composition. When employed, surfactants help maintain dispersion of the rheological modifier and the contrast agent in the solvent. Surfactants also impede the interaction between the rheological modifier and other components of the system. This allows for more

fully developed rheologically-modified systems.

When surfactants are employed, a preferred biocompatible rheologically-modified composition comprises from 1 to 12 weight percent of biocompatible polymer, from 20 to 55 weight percent of a contrast agent, preferably 37 to 40 weight percent of contrast agent, from 1 to 12 percent silica, all based upon total weight of composition and from 10 to 20 weight percent of surfactant, based upon the weight of silica and the remaining weight percent biocompatible solvent.

Bridging molecules may also be employed in the biocompatible rheologically-modified compositions of the instant invention. When employed, bridging molecules act as a dispersion modifier for the rheological modifiers.

When bridging molecules are employed, a preferred biocompatible rheologically-modified composition comprises from 1 to 12 weight percent of biocompatible polymer, from 20 to 55 weight percent of a contrast agent, preferably 37 to 40 weight percent of contrast agent, from 1 to 12 percent silica, all based upon total weight of composition and from 10 to 30 weight percent of bridging molecule, based upon the weight of silica and the remaining weight percent biocompatible solvent.

## Methods

As illustrated in Example 4, the compositions described above can be employed in methods for the catheter assisted intra-vascular embolization of mammalian blood vessels. These methods are employed at intra-vascular sites wherein preferably blood flow is attenuated, but not arrested. Attenuation of blood flow arises by placement of the catheter into the vascular site, wherein blood flow there through is reduced. For example, a microballoon may be employed to attenuate blood flow.

In these methods, a sufficient amount of the biocompatible rheologically-modified composition is introduced into the vascular site via, for example, a catheter under fluoroscopy so that upon precipitation of the polymer, the vascular site is embolized. The particular amount of composition employed is dictated by the total volume of the vasculature to be embolized, the concentration of polymer in the composition, the rate of precipitation (solids formation) of the polymer, etc. Such factors are well within the skill of the art.

In the catheter delivery methods described herein, a small diameter medical catheter (*i.e.*, microcatheter) having a diameter typically from 1 mm to 3 mm is employed. The particular catheter employed is not critical, provided that catheter components are compatible with the composition (*i.e.*, the catheter components will not readily degrade in the composition). In this regard, it is preferred to use polyethylene, or even more preferably PTFE in the catheter components because of its inertness in the presence of the compositions described herein. Other materials compatible with the compositions can be readily determined by the skilled artisan and include, for example, other polyolefins, fluoropolymers (*e.g.*, polytetrafluoroethylene, perfluoroalkoxy resin, fluorinated ethylene propylene polymers, etc.), silicone, etc. The specific polymers employed for contact with the composition are selected based on stability in the presence of the solvent and preferably upon lubricious properties.

## Devices for Delivery

The compositions may be packaged for uses targeted at specific treatment procedures. Accordingly, kits of parts may be provided, with the contents of the kit depending on the particular application, and on factors such as the size, weight, and other characteristics of the patient. As shown in Example 5, these kits can include a variety of devices to assist in successful delivery of the compositions.

The kit of parts would for example include the composition which may be dispensing in a sealed vial or sealed tube. The quantity of composition would be at least enough to perform the particular procedure, and enough surplus to cover inadvertent waste. In addition, a separate quantity of biocompatible solvent, of the same or different makeup from the solvent in the composition, may be provided, for example to flush and prime the delivery system. Non-particulate embolization-enhancing devices or agents may also be provided, such as coils, and so forth. Further, the kit may contain one or more delivery devices, such as a syringe and/or a catheter or microcatheter. The syringe may be preloaded with a quantity of the composition.

It is also contemplated that the composition may be used in conjunction with other procedures, such as repairing or replacing damaged vessels using vascular prostheses, such as for example endovascular prostheses. The composition in such cases may be used to help fuse the prostheses in place and prevent leaks due to incomplete sealing. When intended for such application, the kit of parts may include one or more such prostheses. Of course, as with other applications, directions for use, including for example cautions, warnings, indications, counter-indications, and bibliographies, may also be provided with the kit of parts.

## Utility

The compositions and methods described herein are useful in embolizing mammalian blood vessels and thus

can be used to prevent/control bleeding (*e.g.*, organ bleeding, gastrointestinal bleeding, vascular bleeding, bleeding associated with an aneurysm or an AVM) or to ablate diseased tissue (*e.g.*, tumors, etc.). Accordingly, the invention finds use in human and other mammalian subjects requiring embolization of blood vessels.

It is contemplated that the compositions can be employed as a carrier for a compatible, pharmaceutically-active compound wherein this compound is delivered *in vivo* for subsequent release. Such compounds include by way of example only antibiotics, antiinflammatory agents, chemotherapeutic agents, anti-angiogenic agent, radioactive agents, growth factors and the like.

These composition can also be used in tissue bulking including sphincter bulking, peri-uretheral tissue bulking, soft-tissue augmentation as described in U.S. Patent Nos. 6,231,613; 6,238,335; 6,595,910; and 6,569,417.

The following examples are set forth to illustrate the claimed invention.

## EXAMPLES

Unless otherwise stated, all temperatures are in degrees Celsius. Also, in these examples and elsewhere, the following abbreviations have the following meanings:

| | |
|---|---|
| μm = | micron |
| avg. = | average |
| cc = | cubic centimeter (equal to 1 milliliter) |
| cP = | centipoise |
| cSt = | centistoke |
| D = | shear rate (1/S) |
| DMSO = | dimethylsulfoxide |
| EH5 silica = | fumed silica having a surface area of approximately 380 $m^2/g$ (BET) (available from Cabot Corp., Tuscola, IL., USA) |
| Eta (Greek letter, η) = | apparent viscosity (Pa*s) |
| EVOH = | ethylene vinyl alcohol copolymer |
| Formula K = | 19% EVOH (wt/vol DMSO) or 8.21 % EVOH (wt/final wt);<br>38% (wt/final wt) Ta; and<br>6.175% EH5 silica (wt/final wt) |
| ft = | feet |
| g = | gram |
| kg = | kilogram |
| lb. = | pound |
| m = | meter |
| M5 silica = | fumed silica having a surface area of approximately 200 $m^2/g$ (BET) (available from Cabot Corp., Tuscola, IL., USA) |
| min = | minute |
| ml = | milliliter |

| | |
|---|---|
| mm = | millimeter |
| PA = | Parent artery |
| PAO = | Parent artery occlusion |
| ppm = | parts per million |
| psi = | pound per square inch |
| RPM = | revolutions per minute |
| s = | second |
| Ta = | tantalum |
| Tau (Greek letter, $\tau$) = | shear stress (Pa) |
| TS-610 silica = | fumed silica having a surface area of approximately 125 $m^2/g$ (BET) (available from Cabot Corp., Tuscola, IL., USA) |
| TS-720 silica = | fumed silica having a surface area of approximately 115 $m^2/g$ (BET) (available from Cabot Corp., Tuscola, IL., USA) |
| wt/final wt = | weight per final weight of composition |
| wt/vol DMSO = | weight per volume of DMSO |
| $\pi$g/g = | parts per million |

## Equipment

Unless otherwise indicated, the following equipment was employed in the examples below.

The mixer employed in the experiments was a Morehouse-Cowles (Fullerton, CA) Model CM-100 Lab Disperser with optical encoder and feedback system to maintain constant RPM under variable load. The mixer had a 1.25 inch diameter hi-vane impeller Cowles blade in a 250 ml or 500 ml beaker. There was approximately a 2 to 1 or 3 to 1 ratio of beaker diameter to blade diameter respectively. The blade height, measured from about the bottom of the beaker is one half to one full blade diameter.

In order to obtain viscosity measurements, a Brookfield Engineering (Middleboro, MA) R/S-CPS Rheometer with RS232 interface to Windows-based PC running Brookfield Rheocalc v2.7 software. This is a cone and plate system, with two cones employed. One cone was 50 mm with 1° angle and the other cone was 75 mm with a 1° angle. There was also a Brookfield TC-501 water bath employed for temperature control to the R/S.

## Compositions

In the following examples and procedures, the DMSO is USP grade. The tantalum is Q2 Grade NRC Capacitor grade tantalum metal powder from H.C. Starck (Newton, MA).

## Example 1

## Preparation of Compositions

This example evaluated the density, precipitation and cytotoxicity of non-sterilized polymeric embolic compositions comprising silica as a rheological modifier as compared to non-sterilized embolic composition not containing fumed silica.

Two different rheologically-modified polymeric embolic compositions were evaluated relative to two conventional polymeric embolic compositions. Specifically, two conventional embolic compositions were prepared (based on 100 ml of DMSO) as follows:

**Composition A:**

1) 20 g EVOH (48 percent ethylene-average molecular weight of approximately 100,000);
2) 82 g tantalum; and
3) 100 mL DMSO.

**Composition B:**

1) 30 g EVOH (48 percent ethylene-average molecular weight of approximately 100,000);
2) 123 g tantalum; and
3) 100 mL DMSO.

The DMSO and the EVOH were combined and the resulting composition was covered and heated to 55 ±5°C for 1.0 hours while stirring the composition. The heating was continued at the indicated temperature until all of the EVOH was dissolved. In a stirred vessel, tantalum powder was added to the EVOH and DMSO composition over a period of 30 minutes and stirring was continued for another 20 minutes to ensure homogeneity.

Two rheologically-modified polymeric embolic compositions were prepared in a manner similar to that described above with the exception that each of the components was treated in the manner set forth in Scheme 1 prior to addition and further, after addition of the tantalum, silica was added to the composition under ambient conditions and an argon atmosphere. Each composition was initially stirred at low RPM (less than 1000 RPM) to wet the surface of the silica. Once wetted, the stir rate was increased to a peripheral tip speed of about 20 m/sec. This tip speed was maintained until no granular material was evidenced in composition. This procedure was maintained for 5 minutes and any granular material adhering to the vessel walls was scraped into the fluid composition. Mixing as per above was then continued for another 5 minutes. This tip speed was maintained until no granular material was evidenced in composition.

The blade was positioned between 0.5 and 1 blade diameter above the bottom of the mixing vessel. The diameter of the mixing vessel was 2 to 3 times the diameter of the mixing blade.

Upon completion of mixing of the silica-containing embolic compositions, the compositions comprised the following (based on 100 mL of DMSO)

**Composition C:**

1) 11 g EVOH (48 percent ethylene-average molecular weight of approximately 100,000);
2) 82 g tantalum;
3) 100 mL DMSO; and
4) 14 g M5 silica.

**Composition D:**

1) 11 g EVOH (48 percent ethylene-average molecular weight of approximately 100,000);
2) 82 g tantalum;
3) 100 mL DMSO; and
4) 14 g EH5 silica.

Densities were determined by conventional means.

A portion of each of the compositions was precipitated in saline. The resulting precipitate was washed twice with saline and then employed in the art recognized ISO-10993 cytotoxicity testing.

Table 1 lists the results of this evaluation.

| ***Sample*** | ***Density (g/ml)*** | ***Cytotoxicity Result*** |
|---|---|---|
| Composition C | 1.764 | pass |
| Composition D | 1.778 | pass |
| Composition A | 1.73 ± 0.10 | pass |
| Composition B | 1.971 | pass |

**Example 2**

**Mixing Parameters of Fumed Silica Embolic Compositions**

Mixing parameters were studied.

FIG. 1 illustrates non-sterile and sterile viscosities of the composition D of Example 1 versus shear rate. The composition was 10 % EVOH (wt/vol DMSO), 38 % Ta (wt/final wt), and 7% EH5 silica (wt/final wt) and was mixed with a 1.25" Cowles Blade in a 500 ml beaker at the indicated RPM. FIG. 1 shows that 11,000 RPMs for 10 min produced a non-sterile fluid that was high viscosity at low shear rate and low viscosity at high shear rate (i.e. shear thinning). Upon sterilization, the material shows an increase in high and low shear viscosity

**Quantification of rheologically-modified Composition Properties**

Using the Brookfield R/S-CPS Rheometer, non-sterile formulations were analyzed as in FIG.1, measuring viscosity and shear stress against shear rate.

The shear stress data were plotted using the method of Casson (Casson (1959) Rheology of Dispersed Systems. Pergamon, NY). The Casson equation (see equation 1) gives a straight line with a y-intercept that is the square root of the fluid's yield stress ($K_0$) and a slope ($K_1$) that represents the fluid viscosity at infinite shear rate.

$$\textit{Equation 1} \qquad \sqrt{\tau} = K_o + K_1\sqrt{\gamma}$$

The viscosity data were plotted using a Power Law method (Braun & Rosen (2000) Rheology Modifiers Handbook: practical use and application. William Andrew Publishing , NY). This method often gives data that can be separated into linear segments that are analyzed separately (see Equation 2).

$$\textit{Equation 2} \qquad \ln\eta = \ln K_I + K_2 \ln\gamma$$

FIGs. 7A and 7B show various viscosity property data for various compositions of this invention analyzed by the Casson and Power Law methods, respectively. FIG. 7A shows the formulations in a Casson plot. FIG. 7B shows the formulations in a Power Law method.

Based on the above analysis, four rheologically-modified compositions employing the following ratios of amorphous fumed silica (silica) as the rheological modifier and ethylene vinyl alcohol copolymer (EVOH) as the biocompatible polymer were determined to have the best balance of yield stress (Casson plot in FIG. 7A) and viscosity at rest and shear thinning (Power Law plot in FIG. 7B), with K being the best:

Formula I: 6.25 % silica (wt/final wt) and 7.83 % EVOH (wt/final wt)
Formula J: 6.28 % silica (wt/final wt) and 7.41 % EVOH (wt/final wt)
Formula K: 6.175 % silica (wt/final wt) and 8.21 % EVOH (wt/final wt)
Formula L: 6.38 % silica (wt/final wt) and 6.98 % EVOH (wt/final wt)

**Example 3**

**Formulation Optimization**

The purpose of this testing was to evaluate and rank the *in vitro*, aneurysm embolization performance of several prior art formulations and formulations of rheologically-modified composition in order to optimize the formulation. All embolizations were performed in silicone lateral wall aneurysm models. The primary focus was on determining the ability of the formulations to effectively and consistently fill and model the neck of the aneurysms in a controllable fashion.

Multiple formulations were created with variable parameters of their main constituents (*e.g.*, % EVOH (wt/vol DMSO) and % silica (wt/final wt)). These formulations were evaluated numerically on observed effects during embolization.

The numerical results were then compiled and analyzed. The analysis was used to optimize the combination of input variables (% silica/ % EVOH) versus "Degree of control at the neck" primarily.

**Results and Discussion**

FIG. 6 illustrates a main effects plot showing contributing curves of the input variables (wt/final wt) for control at the neck. The horizontal line represents the highest average performance generated for the neck control response variable. As can be seen, there was a optimal addition of silica, in terms of control of the leakage of composition out of the neck. Similarly, control could be optimized for the amount of polymer. This proves that the control of silica addition and the proper selection of silica and polymer levels can produce a superior embolizing composition. Confirmatory tests produced results which confirmed that Formula K, which followed this optimization, gave virtually no leakage at the neck prior to aneurysm fill.
As noted, Formula K provides the optimal combination of precipitation and flow characteristics yielding the desired control at the neck

**In vitro Aneurysm - Failure Modes**

Silicone aneurysms were embolized with Composition A, as described in Example 1, and Formula K to compare quantitatively the volume of material that produces a parent artery protrusion. The volume that completely fills the aneurysm was measured and then the volume that creates a parent artery protrusion was measured. The ratio of volume that fills the aneurysm versus the volume that create parent artery protrusion is a measure of the material's ability to fill the aneurysm but not leak from the aneurysm. After testing Formula K against a known composition, it was confirmed that Formula K illustrates the optimal combination of precipitation and flow characteristics yielding the desired control at the neck.
The material was also tested for stability after aging. The results indicated that the there was no change in the composition's viscosity properties over time.

**Example 4**

**In Vivo Confirmation**

**Methods**

In general, all *in vivo* embolizations were performed per the procedure described in the canine model below. The performance was measured using the same evaluation system described in the previous example.
A 10-15 kg mongrel dog was anesthetized. Under sterile conditions and with the aid of an operating microscope, an experimental aneurysm was surgically created in the carotid artery using a jugular vein pouch, employing the method of German et al. After about one week, the aneurysm was embolized with a rheologically-modified composition.
Specifically, the femoral arteries are accessed by cut down and introducers and 7 Fr guiding catheters were placed.
For deposition of the rheologically-modified composition, a micro catheter (*e.g.*, Micro Therapeutics, Inc. Titan, with guide wire) was placed through the guiding catheter and was positioned under fluoroscopic guidance so that the catheter tip was in the aneurysmal sac. A microballoon catheter (4-5 mm balloon) was placed in the carotid artery proximal to the aneurysm. Position was confirmed with injection of liquid contrast agent. The balloon was inflated to slow or arrest blood flow to prevent displacement of the rheologically-modified composition comprising fumed silica during injection.
Approximately 0.3 to 1 cc of sterilized rheologically-modified composition, as described in Example 3, was injected into the aneurysm over about 30 minutes to fill the aneurysm space. Care was given not to overfill the aneurysm and block the parent artery with polymer. Filling was easily visualized with fluoroscopy due to the presence of contrast agent in the polymer composition. After about 10 minutes, the polymer was fully precipitated and the catheters was removed from the artery.
Sagittal gross pathology sections of the embolization using compositions of this invention were compared to subjects that were embolized using Composition A, not of the invention, as described in Example 1, illustrate the smooth and complete fill of the rheologically-modified composition. Results of the comparison are presented in FIGs. 8A and 8B.
The above tests provided a very positive in *vivo* confirmation of Formula K with no significant parent artery protrusion and high scores in the "control at the neck" response variable.

**Example 5**

**Delivery System Components Development**

During the development of the rheologically-modified composition, it was determined that the high shear viscosity of the Formula K is approximately 3000-4000 cP. Because of the material's high viscosity, it became desirable to modify some of the delivery system components and to incorporate others in order to handle the increased pressure demands of the material during injection. Out of this discovery, new components, evolved as follows:

These improved components, which may be used individually in combination with conventional syringe and catheter components, or together as will be described, include a vented syringe barrel, an improved connector for coupling a syringe barrel to a tube of material to be injected, a coupling for joining a syringe to a catheter and an improved mechanism, called a "Quick Stop" for easily and repeatedly engaging a lead screw mechanism to extrude the viscous composition out of the syringe and disengaging the lead screw to halt the flow of composition.

Turning to FIG. 9, a combination of components making up a system 190 suitable for loading composition into a syringe barrel is shown in an exploded view. System 190 includes a syringe barrel 192. As illustrated in more detail in FIGs.13 and 14, syringe barrel 192 includes a distal delivery orifice 232 equipped with a male luer fitting 234. The proximal end includes an orifice 236 and a flange or handle 238. Flange 238 is typically a flat-sided oval having a width "Wf'. This width will come into play when the syringe is used in conjunction with the Quick Stop mechanism as will be described hereinafter.

Combination 190 includes a syringe interface 194. As shown in FIGs. 11 and 12 this interface includes a proximal orifice 212 which includes threads 214 and standard luer taper 222 of a female luer fitting to sealably engage the male luer fitting on syringe barrel 192. Interface 194 also included as distal orifice 224. Orifice 224 includes internal threads 230 which are sized to correspond to match the external threads 196 present on tube 198. Tube 198 is constructed of a flexible material such as plastic or aluminum and has a orifice 202 which is typically covered with a frangible seal (not specifically shown) stretched over it. Interface 194 includes a hollow tapered barb 226 with internal opening 228 in fluid communication with orifice 212. In use, interface 194 is typically screwed into syringe body 192 and screwed onto tube 198. As interface 194 is tightened onto tube 198, via threads 196, this causes tapered barb 226 to contact and pierce the seal on tube 198 and establish a fluid flow path out of the tube, through the interface and into the syringe barrel. By squeezing tube 198, its contents, for example a relatively viscous embolic composition, can be loaded into the syringe barrel 192.

Syringe barrel 192 is equipped with at least one vent hole 240. Vent hole 240 is provided to allow air to be removed from the syringe barrel and assure a bubble-free fill of the barrel with the viscous embolizing composition or a like viscous injectable. In use, the injectable material is filled into the syringe barrel at least to the vent hole. Then when a plunger, either a conventional plunger or a plunger such as plunger 204 having threads 206 on its shaft 208 is inserted in the proximal end of the syringe body, any air that is trapped between the body of injectable composition and the syringe plunger is exhausted out through the vent hole 240. It will be appreciated that with conventional injectables which are commonly very low viscosity solutions suspensions, there is little need for vent holes. Bubbles which are trapped in a less viscous injectable can be dislodged by tapping on the syringe body and organized at the distal end of the syringe and readily expelled thought the distal orifice. This is very difficult to accomplish, if not virtually impossible, with viscous injectables such as the embolizing compositions of this invention. Accordingly, the vent holes provide a significant advantage.

Turning to FIG. 10 and referring to FIG. 9, as well, an improved system 200 for delivering a viscous injectable, and in particular the viscous embolizing compositions of this invention is shown. The viscous injectable is not shown but would have been loaded into syringe barrel 192 to a volume at least up to the vent holes 240. In use, interface 194 would be removed and replaced by adapter 216. Adapter 216 includes a female luer connector which matches and engages connector 234 on barrel 194. At its other end adaptor 216 is threaded with a thread 220 corresponding to a thread on catheter 242. Adapter 216 sealably joins catheter 242 to syringe barrel 192 and creates a fluid path between them.

System 200 also includes Quick Stop 242. Quick Stop 242 is illustrated in detail in FIGs. 15-19 and its components are shown in FIGs. 20-25. As shown most clearly in FIG. 17, Quick Stop 242 is sized to slide over and engage the flange 238 on syringe barrel 192. As noted previously, syringe body 192 included a flange 238 which has a width Wf. Quick Stop 242 has a slot 244 defined by shoulders 246 and 246' which is somewhat wider than flange 238. Thus, as shown in FIG. 10, when Quick Stop 242 is slid along flange 238 with its lines A-A' and B-B' corresponding to the same lines on the flange 238 , the two parts engage as shown in FIG. 17. As can be seen in FIG. 10, and in more detail in FIGs. 16 and 18, shoulder 246 and 246' not only define a slot in which the syringe barrel 192 is inserted, they also extent inward toward each other and join at region 24. This region limits the insertion of the flange 238 of syringe barrel 194 to a predetermined distance. This predetermined distance is such that threaded hole 250 is axially aligned with barrel 192.

As shown in FIG. 15's perspective top view, hole 250 is contained in base 252. Activator 254 slides back and

forth, relative to base 252, along the same direction that barrel 193 slid into slot 244. FIG. 24 depicts actuator 254 and shows that it has two pairs of inwardly-extending fingers, 256 and 256' and 258 and 258'. These fingers bear upon surfaces of a part known as threaded pincher 260. FIG. 25 shows that 260 has a pivot point 262 and has two wings 264 and 264'which can move together and apart relative to one another. Wings 264 and 264' each contain a portion of a threaded aperture 266 and 266'. When the two wings are brought together they define a nearly complete threaded cylinder which is matched to the thread 208 present on threaded syringe plunger shaft 206. By "nearly complete" it is meant that at least about 280° of the full circle are created and preferably at least 300° and especially at least about 320° degrees. This extent of circle creation is important as it assures that there is a solid grip in the threaded shaft 208 of plunger 204 when the threaded pincher is closed around it and it permits greater forces to be exerted on the plunger 204 to extrude the high viscosity embolizing compositions.

As can be seen most clearly in FIGs. 18 and 19, actuator 254 is moved as far to the left as possible relative to base 252. This has caused fingers 256 and 256' to bear against surfaces 268 and 268' on threaded pincher 260 and this in turn has caused threads 266 and 266' to form a threaded aperture in its "on" or "engagement" mode. If threaded plunger shaft 208 was in place in aperture 250, this would result in threads 266 and 266' engaging the threads 206 on shaft 208. When so engaged, threads 206 on shaft 208 would function as a lead screw when rotated such as by knob 270. This would cause plunger 204 to move inward or outward relative to syringe body 192, depending upon the direction of rotation of knob 270. This permits the gradual and controllable delivery of the viscous composition out of the syringe barrel to the location of use such as through catheter 242. If activator 254 were slid to the right, this would release the pressure or fingers 256 and 256' on surfaces 268 and 268', respectively and would, instead cause fingers 258 and 258' to engage and push apart surfaces 270 and 270' on threaded pincher 360. This would cause the threaded aperture to "open". This would create an opening though which plunger 204 could be inserted. It would also release the grip in threaded plunger shaft 208, if the plunger were in place passing through hole 250 into barrel 192. When this grip is released it would instantaneously release the pressure e on the viscous material in the syringe barrel and halt the material's delivery such as through catheter 242.

As seen most clearly in FIGs. 20 and 23, base 252 and activator 254 may be equipped with one or more detent mechanisms to allow the device to be temporarily locked in the "on" or "engaged" position. This can be accomplished with, for example indents 274 and 274' which engage extensions 276 and 276'. This locking mechanism can prevent inadvertent releases of pressure as might occur if the actuator were permitted to freely move out of the on position.

## Example 5A

### Quick Stop clip for the threaded injector

This is the new cam-slide mechanism that incorporates an approximate 340° thread engagement as opposed to the known Quick Stop as described in United States Patent Application Publication No. 2003-0055386, which only engaged approximately 220° of the plunger thread. In addition, this new mechanism reduces, significantly, the amount of force necessary to engage and disengage the plunger.

The static pressure capability of the new clip was tested and the average peak pressure (psi) was 2,731.

The force to engage and disengage threads was also tested compared with the old clip. When completing 10 cycles, the average force (Ib.) was to engage the threads was 3.45, as compared to 8.48 for the old clip. To disengage the threads, an average force of 3.28 was required, compared to 8.18 for the old clip.

Based on the above results, the new Quick Stop is a significant improvement over the prior mechanism. The force required to engage and disengage the new clip is less than half the original. In addition, the pressure capability of the new clip has been increased by approximately 1000 psi.

Schematic drawings of the Quick Stop mechanism are shown in FIG. 10 and FIG. 15 through FIG. 25.

## Example 5B

### Stainless steel syringe-to-catheter interface

The peak pressure of the interface at a given flow rate of 0.1 ml/min, 0.2 ml/min., 0.3 ml/min, and 0.5 ml/min was tested. The average pressures (psi) were as follows: at 0.1 ml/min, 948 psi; at 0.2 ml/min, 1520 psi; 0.3ml/min., 1829; and 0.5 ml/min, 1897.

Based on the above results, the new interface fitting 216 is a significant improvement over the current HD injector connection. The pressure capability of the new fitting has been increased by approximately 400 psi.

**Example 5C**

**Modified threaded plunger**

The modified plunger (204 in the drawings) employs a change to the diameter of the piston head before and after the O-rings for the purpose of increasing its pressure capability. The prior design can withstand approximately 1700-1900 psi before the O-rings would begin to peel back and fail. By increasing the surface area (diameter) of the leading head on the piston and by increasing the diameter of the piston body behind each o-ring, the pressure capability of the plunger could be significantly increased. When testing the static pressure capacity of the new Quick Stop clip, the modified plunger was used.

**Example 5D**

**Puncture cap with standard luer interface**

With the new aluminum tube packaging described herein comes the need to be able to puncture the tube membrane and transfer the material to the delivery syringe. To meet this need, a suitable puncture cap with luer interface 194 was designed. This device is a small cap. As described above, one end screws onto the tube while creating a burrless puncture hole in the membrane and the other end has a ISO luer fitting that can be fitted to the tip of the threaded injector for the composition transfer.

**Functionality Test**

The new cap effectively and cleanly punctured the tube membrane and connected to the delivery syringe with ease as designed.

**Example 7**

**E-Beam Sterilization of Formula K in an Aluminum Tube**

Formula K, a described above, was injected into 3 mL aluminum tubes (30 in all) also as described above. The 30 tubes were divided into three groups of 10, 8 subject to e-beam sterilization and two serving as controls. The first group, other than the control samples, was subjected to e-beam sterilization at a total dosing of 20 kGy. The second group, other than the control samples, was subjected to e-beam sterilization at a total dosing of 40 kGy. The third group, other than the control samples, was subjected to e-beam sterilization at a total dosing of 60 kGy.

It was observed that there was little change in the rheological profile of the e-beam sterilized compositions relative to control. Contrarily, the heat sterilized composition exhibits significant deviation in its rheological properties as compared to control.

The table below illustrates the area between the two curves measuring shear stress at increasing and decreasing shear rates measured at from 0 to 250 $s^{-1}$ for each of these compositions:

| | |
|---|---|
| Control | 2,248 Pa/sec |
| E-beam sterilized | 10,684 Pa/sec |
| Heat Sterilized | 36,880 Pa/sec |

The above data demonstrates that the e-beam sterilized compositions of this invention retain pseudo-plastic properties similar to control whereas the heat sterilized compositions did not.

From the foregoing description, various modifications and changes in the composition and method will occur to those skilled in the art.

**Claims**

**1.** A composition comprising:

(1) a biocompatible polymer insoluble in blood or other body fluid,

(2) a biocompatible solvent that is capable of solubilizing the biocompatible polymer and is miscible in blood or other body fluid,
(3) a biocompatible contrast agent, and
(4) a sufficient amount of fumed silica to act as a rheological modifier in the composition and to impart a shear thinning index to the composition, measured at shear rates of 1 $s^{-1}$ and 10 $s^{-1}$ at 37°C, of at least 4.

**2.** The composition according to Claim 1 having a high viscosity at low shear conditions as defined by a viscosity of greater than 25,000 cP at 37°C at a shear rate of 0.24 $s^{-1}$ and a viscosity of less than 5000 cP at 37°C when a shear rate of at least 100 $s^{-1}$ is applied.

**3.** The composition according to Claim 1 having an intermediate viscosity at low shear conditions as defined by a viscosity of greater than 4,000 cP at 37°C at a shear rate of 0.24 $s^{-1}$ and a viscosity of less than 2000 cP at 37°C when a shear rate of at least 100 $s^{-1}$ is applied.

**4.** The composition according to Claim 2 or 3, wherein said composition is an embolic composition used to embolize a vascular site for treatment of one or more of an aneurysm, arteriovenous fistulae, and uncontrolled bleeding.

**5.** The composition according to Claim 2 , wherein the biocompatible polymer and fumed silica are present in the composition in a ratio of from 2.6 to 1 to 3.6 to 1 on a weight of polymer per volume of solution to weight of silica per total weight basis.

**6.** The composition according to Claim 5 wherein said ratio is from 3.0 to 1 to 3.2 to 1.

**7.** The composition according to Claim 5, wherein said ratio is 3.1 to 1.

**8.** The composition of Claim 1 having a low viscosity at low shear conditions as defined by a viscosity of greater than 2,000 cP at 37°C at a shear rate of 0.24 $s^{-1}$ and a viscosity of less than 500 cP at 37°C when a shear rate of at least 100 $s^{-1}$ is applied.

**9.** The composition as in Claim 8, wherein said composition is an embolic composition used to embolize a vascular site for the treatment of one or more of arteriovenous malformations, tumors, and uncontrolled bleeding.

**10.** The composition according to Claim 1, wherein the biocompatible polymer is selected from the group consisting of cellulose acetate butyrate, cellulose diacetate, polymethyl methacrylate, polyvinyl acetate, copolymers of urethane and acrylates, ethylene vinyl alcohol, and mixtures thereof.

**11.** The composition according to Claim 10, wherein the biocompatible polymer is ethylene vinyl alcohol.

**12.** The composition according to Claim 1, wherein the biocompatible solvent is selected from the group consisting of ethyl alcohol, ethyl lactate, acetone, dimethylsulfoxide, and mixtures thereof.

**13.** The composition according to Claim 12, wherein the biocompatible solvent is dimethylsulfoxide.

**14.** The composition according to Claim 1, wherein the biocompatible contrast agent is selected from the group consisting of tantalum, tantalum oxide, tungsten, barium sulfate, and mixtures thereof.

**15.** The composition according to Claim 14, wherein the contrast agent is tantalum.

**16.** The composition according to Claim 1, wherein the composition further comprises a bridging molecule.

**17.** The composition according to Claim 12, wherein the bridging molecule is a glycol.

**18.** The composition according to Claim 1, wherein the composition further comprises a surfactant.

**19.** The composition according to Claim 1, wherein the biocompatible polymer is ethylene vinyl alcohol, the biocompatible solvent is dimethylsulfoxide, and the biocompatible contrast agent is tantalum.

**20.** The composition according to Claim 19, wherein the ethylene vinyl alcohol and fumed silica are present in the

composition in a ratio of 3.1 to 1 on a weight of polymer per volume of solution to weight of silica per total weight basis.

**21.** A composition suitable for vascular embolization comprising:

ethylene vinyl alcohol 3-9 % weight
tantalum contrast agent 37-40 % weight
DMSO solvent and
fumed silica in an amount to impart a shear thinning index to the composition measured at 1 $s^{-1}$ and 10 $s^{-1}$ at 37°C, of 4.0 to 6.5.

**22.** The composition according to Claim 21 comprising ethylene vinyl alcohol 8.2 % weight, tantalum 38 % weight and fumed silica 6.2 % in DMSO solvent.

**23.** Use of a composition according to Claim 1 to provide a means for embolizing a vascular site in a mammal, wherein the embolizing of the vascular site in said mammal can be achived by delivering via a catheter into the vascular site said composition under conditions wherein the composition forms a precipitate in the vascular site which precipitate embolizes the vascular site.

**24.** The use of Claim 23 wherein said composition is a high viscosity composition according to Claim 2.

**25.** The use according to Claim 24, wherein said vascular site is in need of embolization due to one or more of an aneurysm, arteriovenous fistulae, and uncontrolled bleeding.

**26.** The use according to Claim 23, wherein said composition is an intermediate viscosity composition according to Claim 3.

**27.** The use according to Claim 26, wherein said vascular site is in need of embolization due to one or more of an aneurysm, arteriovenous fistulae, and uncontrolled bleeding.

**28.** The use according to Claim 23, wherein said composition is a low viscosity composition according to Claim 8.

**29.** The use according to Claim 28 wherein the vascular site is in need of embolization due to one or mores of arteriovenous malformations, tumors, and uncontrolled bleeding.

**30.** A kit of parts suitable for use in embolizing a selected vascular site comprising:

a composition of Claim 1 and
a catheter sized and selected to be compatible with the selected vascular site.

**31.** The kit according to Claim 30, wherein the catheter is a microcatheter.

**32.** The kit according to Claim 31, wherein the catheter is suitable for use with a guidewire.

**33.** The kit according to Claim 30, further including a syringe for feeding the composition of claim 1 to the catheter.

**34.** The kit according to Claim 30, further including a vascular prosthesis.

**35.** The kit according to Claim 34, wherein said vascular prosthesis is an endovascular prosthesis.

**36.** The kit according to Claim 30, further including a quantity of a non-particulate agent.

**37.** The kit according to Claim 36, wherein said non-particulate agent is one or more coils.

**38.** The kit according to Claim 30, further including directions for use relating to a treatment procedure.

**39.** The kit according to Claim 38, wherein said treatment procedure comprises embolization of a blood vessel for treating one or more aneurysms or arteriovenous fistulae.

**40.** The kit according to Claim 39, wherein the treatment procedure includes attaching a prosthesis to a blood vessel.

## Patentansprüche

**1.** Zusammensetzung umfassend:

(1) ein biokompatibles Polymer, welches in Blut oder einer anderen Körperflüssigkeit unlöslich ist,
(2) ein biokompatibles Lösungsmittel, welches zur Auflösung des biokompatiblen Polymers in der Lage ist und in Blut oder einer anderen Körperflüssigkeit mischbar ist,
(3) ein biokompatibles Kontrastmittel und,
(4) eine ausreichende Menge an Kieselpulver, welches als ein Rheologie-Modifiziermittel in der Zusammensetzung wirkt und der Zusammensetzung, gemessen bei Scherraten von 1 $s^{-1}$ und 10 $s^{-1}$ bei 37 °C, einen Scherentzähungs-Index von mindestens 4 verleiht.

**2.** Zusammensetzung gemäß Anspruch 1, die eine hohe Viskosität bei niedrigen Scherbedingungen, wie definiert durch eine Viskosität von mehr als 25 000 cP bei 37 °C bei einer Scherrate von 0,24 $s^{-1}$ und eine Viskosität von weniger als 5 000 cP bei 37 °C, wenn eine Scherrate von mindestens 100 $s^{-1}$ zur Anwendung kommt, aufweist.

**3.** Zusammensetzung gemäß Anspruch 1, die eine mittlere Viskosität bei niedrigen Scherbedingungen, wie definiert durch eine Viskosität von mehr als 4 000 cP bei 37 °C bei einer Scherrate von 0,24 $s^{-1}$ und einer Viskosität von weniger als 2 000 cP bei 37 °C, wenn eine Scherrate von mindestens 100 $s^{-1}$ zur Anwendung kommt, aufweist.

**4.** Zusammensetzung gemäß Anspruch 2 oder 3, wobei die Zusammensetzung eine Emboliezusammensetzung ist, die verwendet wird, um eine vaskuläre Stelle zur Behandlung von einem oder mehreren aus einem Aneurysma, einer arteriovenösen Fistel und unkontrolliertem Bluten zu embolisieren.

**5.** Zusammensetzung gemäß Anspruch 2, wobei das biokompatible Polymer und das Kieselpulver in der Zusammensetzung in einem Verhältnis von 2,6 zu 1 bis 3,6 zu 1 auf Basis vom Polymergewicht pro Lösungsvolumen zum Silicagewicht pro Gesamtgewicht vorliegen.

**6.** Zusammensetzung gemäß Anspruch 5, wobei das Verhältnis von 3,0 zu 1 bis 3,2 zu 1 ist.

**7.** Zusammensetzung gemäß Anspruch 5, wobei das Verhältnis von 3,1 zu 1 ist.

**8.** Zusammensetzung gemäß Anspruch 1 mit einer niedrigen Viskosität bei niedrigen Scherbedingungen, wie sie durch eine Viskosität von mehr als 2 000 cP bei 37°C bei einer Scherrate von 0,24 $s^{-1}$ und einer Viskosität von weniger als 500 cP bei 37°C, wenn eine Scherrate von mindestens 100 $s^{-1}$ zur Anwendung kommt, definiert ist.

**9.** Zusammensetzung wie in Anspruch 8, wobei die Zusammensetzung eine Emboliezusammensetzung ist, die verwendet wird, um eine vaskuläre Stelle zur Behandlung von einem oder mehreren aus arteriovenösen Fehlbildungen, Tumoren und unkontrolliertem Bluten zu embolisieren.

**10.** Zusammensetzung gemäß Anspruch 1, wobei das biokompatible Polymer aus der Gruppe gewählt wird, die aus Celluloseacetatbutyrat, Cellulosediacetat, Polymethylmethacrylat, Polyvinylacetat, Copolymeren von Urethan und Acrylaten, Ethylenvinylalkohol und Mischungen davon besteht.

**11.** Zusammensetzung gemäß Anspruch 10, wobei das biokompatible Polymer Ethylenvinylalkohol ist.

**12.** Zusammensetzung gemäß Anspruch 1, wobei das biokompatible Lösungsmittel aus der Gruppe gewählt wird, die aus Ethylalkohol, Ethyllactat, Aceton, Dimethylsulfoxid und Mischungen davon besteht.

**13.** Zusammensetzung gemäß Anspruch 12, wobei das biokompatible Lösungsmittel Dimethylsulfoxid ist.

**14.** Zusammensetzung gemäß Anspruch 1, wobei das biokompatible Kontrastmittel aus der Gruppe gewählt wird, die aus Tantal, Tantaloxid, Wolfram, Bariumsulfat und Mischungen davon besteht.

**15.** Zusammensetzung gemäß Anspruch 14, wobei das Kontrastmittel Tantal ist.

**16.** Zusammensetzung gemäß Anspruch 1, wobei die Zusammensetzung ferner ein Verbrückungsmolekül umfasst.

**17.** Zusammensetzung gemäß Anspruch 12, wobei das Verbrückungsmolekül ein Glycol ist.

**18.** Zusammensetzung gemäß Anspruch 1, wobei die Zusammensetzung ferner ein Surfactant umfasst.

**19.** Zusammensetzung gemäß Anspruch 1, wobei das biokompatible Polymer Ethylenvinylalkohol ist, das biokompatible Lösungsmittel Dimethylsulfoxid ist und das biokompatible Kontrastmittel Tantal ist.

**20.** Zusammensetzung gemäß Anspruch 19, wobei der Ethylenvinylalkohol und das Kieselpulver in der Zusammensetzung in einem Verhältnis von 3,1 zu 1 auf Basis vom Polymergewicht pro Lösungsvolumen zum Silicagewicht pro Gesamtgewicht vorliegen.

**21.** Zusammensetzung, die zur vaskulären Embolisierung geeignet ist, umfassend:

Ethylenvinylalkohol: 3 - 9 Gew.-%
Tantal-Kontrastmittel: 37 - 40 Gew.-%
DMSO-Lösungsmittel und
Kieselpulver in einer Menge, um der Zusammensetzung einen Scherentzähungs-Index, gemessen bei 1 $s^{-1}$ und 10 $s^{-1}$ bei 37 °C, von 4,0 bis 6,5 zu verleihen.

**22.** Zusammensetzung gemäß Anspruch 21, umfassend Ethylenvinylalkohol zu 8,2 Gew.-%, Tantal zu 38 Gew.-% und Kieselpulver zu 6,2 % in DMSO-Lösungsmittel.

**23.** Verwendung einer Zusammensetzung gemäß Anspruch 1, um ein Mittel zur Embolisierung einer vaskulären Stelle in einem Säuger bereitzustellen, wobei die Embolisierung der vaskulären Stelle in dem Säuger erreicht werden kann, indem über einen Katheder in die vaskuläre Stelle die Zusammensetzung unter Bedingungen zugeführt wird, wobei die Zusammensetzung in der vaskulären Stelle ein Präzipitat bildet, wobei das Präzipitat die vaskuläre Stelle embolisiert.

**24.** Verwendung von Anspruch 23, wobei die Zusammensetzung eine Zusammensetzung mit hoher Viskosität gemäß Anspruch 2 ist.

**25.** Verwendung gemäß Anspruch 24, wobei die vaskuläre Stelle einer Embolisierung bedarf auf Grund von einem oder mehreren aus einem Aneurysma, einer arteriovenösen Fistel und unkontrolliertem Bluten.

**26.** Verwendung gemäß Anspruch 23, wobei die Zusammensetzung eine Zusammensetzung mit mittlerer Viskosität gemäß Anspruch 3 ist.

**27.** Verwendung gemäß Anspruch 26, wobei die vaskuläre Stelle einer Embolisierung bedarf auf Grund von einem oder mehreren aus einem Aneurysma, einer arteriovenösen Fistel und unkontrolliertem Bluten.

**28.** Verwendung gemäß Anspruch 23, wobei die Zusammensetzung eine Zusammensetzung mit niedriger Viskosität gemäß Anspruch 8 ist.

**29.** Verwendung gemäß Anspruch 28, wobei die vaskuläre Stelle einer Embolisierung bedarf auf Grund von einem oder mehreren aus arteriovenösen Fehlbildungen, Tumoren und unkontrolliertem Bluten.

**30.** Kit von Teilen, welches zur Verwendung bei der Embolisierung einer ausgewählten vaskulären Stelle geeignet ist, umfassend:

eine Zusammensetzung von Anspruch 1 und
einen Katheder, der so bemessen ist und gewählt ist, um mit der ausgewählten vaskulären Stelle kompatibel zu sein.

**31.** Kit gemäß Anspruch 30, wobei der Katheder ein Mikrokatheder ist.

**32.** Kit gemäß Anspruch 31, wobei der Katheder zur Verwendung mit einem Führungsdraht geeignet ist.

**33.** Kit gemäß Anspruch 30, ferner eine Spritze zur Zuführung der Zusammensetzung von Anspruch 1 zum Katheder einschließend.

**34.** Kit gemäß Anspruch 30, welches ferner eine vaskuläre Prothese einschließt.

**35.** Kit gemäß Anspruch 34, wobei die vaskuläre Prothese eine endovaskuläre Prothese ist.

**36.** Kit gemäß Anspruch 30, ferner eine Menge von einem nichtteilchenförmigen Mittel einschließend.

**37.** Kit gemäß Anspruch 36, wobei das nichtteilchenförmige Mittel eine oder mehrere Coils bzw. Spiralen ist.

**38.** Kit gemäß Anspruch 30, das ferner Anweisungen zur Verwendung in Bezug auf eine Behandlungsprozedur einschließt.

**39.** Kit gemäß Anspruch 38, wobei die Behandlungsprozedur die Embolisierung eines Blutgefäßes zur Behandlung von einem oder mehreren Aneurysmen oder arteriovenösen Fisteln umfasst.

**40.** Kit gemäß Anspruch 39, wobei die Behandlungsprozedur die Anbringung einer Prothese an ein Blutgefäß einschließt.

## Revendications

**1.** Composition comprenant :

(1) un polymère biocompatible insoluble dans le sang ou autre fluide corporel,
(2) un solvant biocompatible qui est capable de solubiliser le polymère biocompatible et qui est miscible dans le sang ou autre fluide corporel,
(3) un agent de contraste biocompatible, et
(4) une quantité suffisante de silice sublimée pour agir en tant que modificateur rhéologique dans la composition et pour conférer un indice de fluidification par cisaillement à la composition, mesuré pour des taux de cisaillement de 1 $s^{-1}$ et 10 $s^{-1}$ à 37 °C, d'au moins 4.

**2.** Composition selon la revendication 1, possédant une viscosité élevée pour des conditions de faible cisaillement ainsi qu'il est défini par une viscosité supérieure à 25 000 cP à 37 °C pour un taux de cisaillement de 0,24 $s^{-1}$ et une viscosité inférieure à 5 000 cP à 37 °C quand un taux de cisaillement d'au moins 100 $s^{-1}$ est appliqué.

**3.** Composition selon la revendication 1, possédant une viscosité intermédiaire pour des conditions de faible cisaillement ainsi qu'il est défini par une viscosité supérieure à 4 000 cP à 37 °C pour un taux de cisaillement de 0,24 $s^{-1}$ et une viscosité inférieure à 2 000 cP à 37 °C quand un taux de cisaillement d'au moins 100 $s^{-1}$ est appliqué.

**4.** Composition selon la revendication 2 ou 3, dans laquelle ladite composition est une composition embolique utilisée pour emboliser un site vasculaire pour le traitement de l'un ou plusieurs parmi un anévrysme, une fistule artérioveneuse et un saignement incontrôlé.

**5.** Composition selon la revendication 2, dans laquelle le polymère biocompatible et la silice sublimée sont présents dans la composition à raison d'un rapport allant de 2,6 à 1 à 3,6 à 1 sur la base d'un poids de polymère par volume de solution à un poids de silice par poids total.

**6.** Composition selon la revendication 5, dans laquelle ledit rapport se situe de 3,0 à 1 à 3,2 à 1.

**7.** Composition selon la revendication 5, dans laquelle ledit rapport est de 3,1 à 1.

**8.** Composition selon la revendication 1, possédant une faible viscosité pour des conditions de faible cisaillement ainsi qu'il est défini par une viscosité supérieure à 2 000 cP à 37 °C pour un taux de cisaillement de 0,24 $s^{-1}$ et une viscosité inférieure à 500 cP à 37 °C quand un taux de cisaillement d'au moins 100 $s^{-1}$ est appliqué.

**9.** Composition telle que selon la revendication 8, dans laquelle ladite composition est une composition embolique utilisée pour emboliser un site vasculaire pour le traitement de l'un ou plusieurs parmi des malformations artériovéneuses, des tumeurs et un saignement incontrôlé.

**10.** Composition selon la revendication 1, dans laquelle le polymère biocompatible est choisi parmi le groupe consistant en un acétobutyrate de cellulose, un diacétate de cellulose, un polyméthacrylate de méthyle, un polyacétate de vinyle, des copolymères d'uréthane et d'acrylates, un éthylène alcool vinylique et des mélanges de ceux-ci.

**11.** Composition selon la revendication 10, dans laquelle le polymère biocompatible est un éthylène alcool vinylique.

**12.** Composition selon la revendication 1, dans laquelle le solvant biocompatible et choisi parmi le groupe consistant en l'alcool éthylique, le lactate d'éthyle, l'acétone, le diméthylsulfoxyde et des mélanges de ceux-ci.

**13.** Composition selon la revendication 12, dans laquelle le solvant biocompatible est le diméthylsulfoxyde.

**14.** Composition selon la revendication 1, dans laquelle l'agent de contraste biocompatible est choisi parmi le groupe consistant en le tantale, l'oxyde de tantale, le tungstène, le sulfate de baryum et des mélanges de ceux-ci.

**15.** Composition selon la revendication 14, dans laquelle l'agent de contraste est le tantale.

**16.** Composition selon la revendication 1, dans laquelle la composition comprend en outre une molécule de pontage.

**17.** Composition selon la revendication 12, dans laquelle la molécule de pontage est un glycol.

**18.** Composition selon la revendication 1, dans laquelle la composition comprend en outre un agent tensioactif.

**19.** Composition selon la revendication 1, dans laquelle le polymère biocompatible est un éthylène alcool vinylique, le solvant biocompatible est le diméthylsulfoxyde, et l'agent de contraste biocompatible est le tantale.

**20.** Composition selon la revendication 19, dans laquelle l'éthylène alcool vinylique et la silice sublimée sont présents dans la composition à raison d'un rapport de 3,1 à 1 sur la base d'un poids de polymère par volume de solution à un poids de silice par poids total.

**21.** Composition appropriée pour une embolisation vasculaire comprenant :

3 à 9 % en poids d'éthylène alcool vinylique
37 à 40 % en poids d'agent de contraste tantale du solvant DMSO et
de la silice sublimée à raison d'une quantité pour conférer un indice de fluidification par cisaillement à la composition mesuré à 1 $s^{-1}$ et 10 $s^{-1}$ à 37 °C, de 4,0 à 6,5.

**22.** Composition selon la revendication 21, comprenant 8,2 % en poids d'éthylène alcool vinylique, 38 % en poids de tantale et 6,2 % en poids de silice sublimée dans du solvant DMSO.

**23.** Utilisation d'une composition selon la revendication 1 pour procurer un moyen destiné à emboliser un site vasculaire chez un mammifère, dans lequel l'embolisation du site vasculaire chez ledit mammifère peut être atteinte en délivrant par l'intermédiaire d'un cathéter dans le site vasculaire ladite composition dans des conditions dans lesquelles la composition forme un précipité dans le site vasculaire, lequel précipité embolise le site vasculaire.

**24.** Utilisation selon la revendication 23, dans laquelle ladite composition est une composition de viscosité élevée selon la revendication 2.

**25.** Utilisation selon la revendication 24, dans laquelle ledit site vasculaire nécessite une embolisation du fait de l'un ou plusieurs parmi un anévrysme, une fistule artérioveineuse et un saignement incontrôlé.

**26.** Utilisation selon la revendication 23, dans laquelle ladite composition est une composition de viscosité intermédiaire selon la revendication 3.

**27.** Utilisation selon la revendication 26, dans laquelle ledit site vasculaire nécessite une embolisation du fait de l'un ou plusieurs parmi un anévrysme, une fistule artérioveineuse et un saignement incontrôlé.

**28.** Utilisation selon la revendication 23, dans laquelle ladite composition est une composition de faible viscosité selon la revendication 8.

**29.** Utilisation selon la revendication 28, dans laquelle ledit site vasculaire nécessite une embolisation du fait de l'un ou plusieurs parmi des malformations artériovéneuses, des tumeurs et un saignement incontrôlé.

**30.** Kit de parties approprié pour une utilisation dans l'embolisation d'un site vasculaire sélectionné comprenant :

une composition selon la revendication 1 et
un cathéter dimensionné et sélectionné pour être compatible avec le site vasculaire sélectionné.

**31.** Kit selon la revendication 30, dans lequel le cathéter est un microcathéter.

**32.** Kit selon la revendication 31, dans lequel le cathéter est approprié pour une utilisation avec un fil guide.

**33.** Kit selon la revendication 30, incluant en outre une seringue destinée à fournir la composition selon la revendication 1 au cathéter.

**34.** Kit selon la revendication 30, incluant en outre une prothèse vasculaire.

**35.** Kit selon la revendication 34, dans lequel ladite prothèse vasculaire est une prothèse endovasculaire.

**36.** Kit selon la revendication 36, incluant en outre une quantité d'un agent non particulaire.

**37.** Kit selon la revendication 36, dans lequel ledit agent non particulaire consiste en une ou plusieurs spirales.

**38.** Kit selon la revendication 30, incluant en outre un mode d'emploi se rapportant à une procédure de traitement.

**39.** Kit selon la revendication 38, dans lequel ladite procédure de traitement comprend l'embolisation d'un vaisseau sanguin pour traiter l'un ou plusieurs parmi un anévrysme ou une fistule artériovéneuse.

**40.** Kit selon la revendication 39, dans lequel la procédure de traitement inclut de fixer une prothèse à un vaisseau sanguin.

FIG. 1

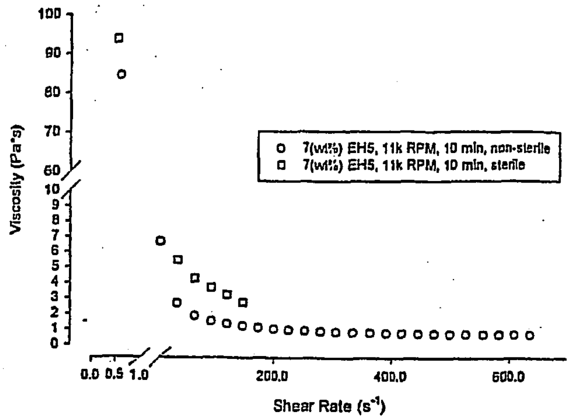


FIG. 2

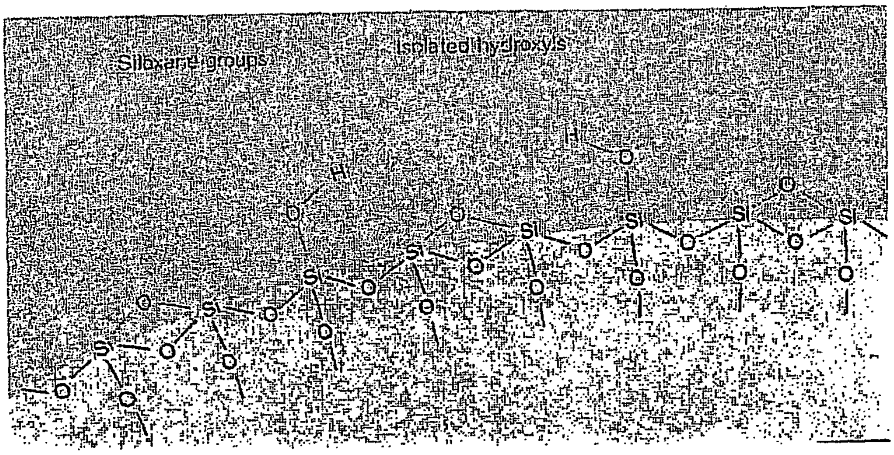


FIG. 3B

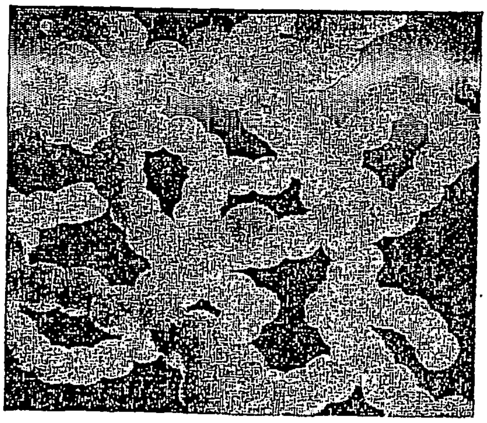

FIG. 3A

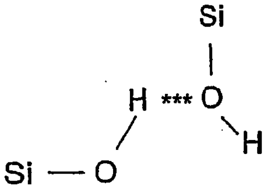

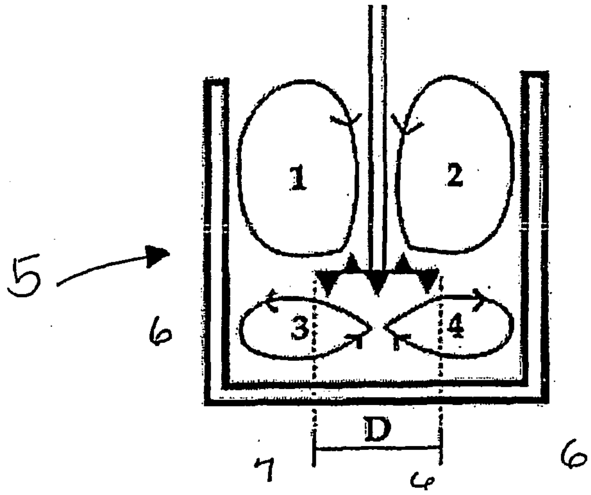
1
2
3
4
5
6
6
7
D

FIG. 4

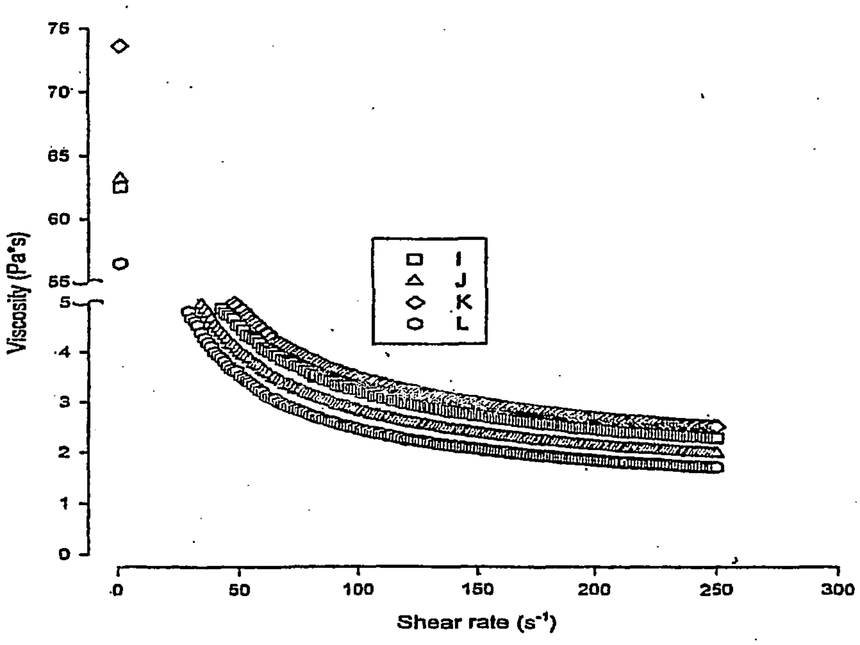
Viscosity (Pa*s)
75
70
65
60
55
5
4
3
2
1
0
0
50
100
150
200
250
300
Shear rate (s-1)
I
J
K
L

FIG. 5

FIG. 6

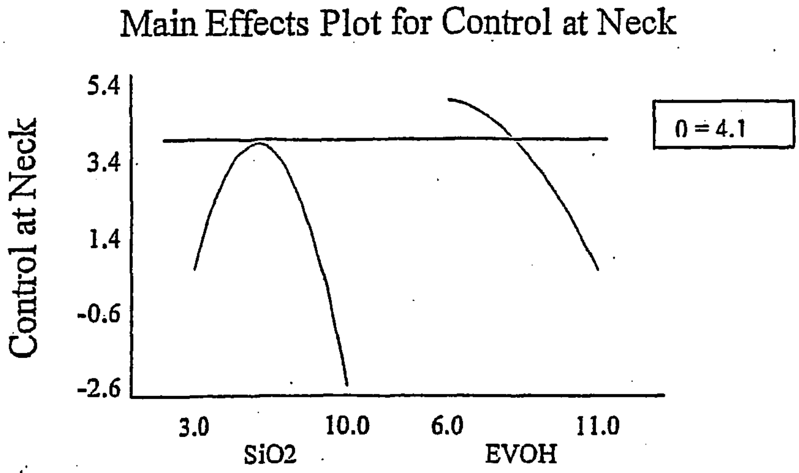


FIG. 7A

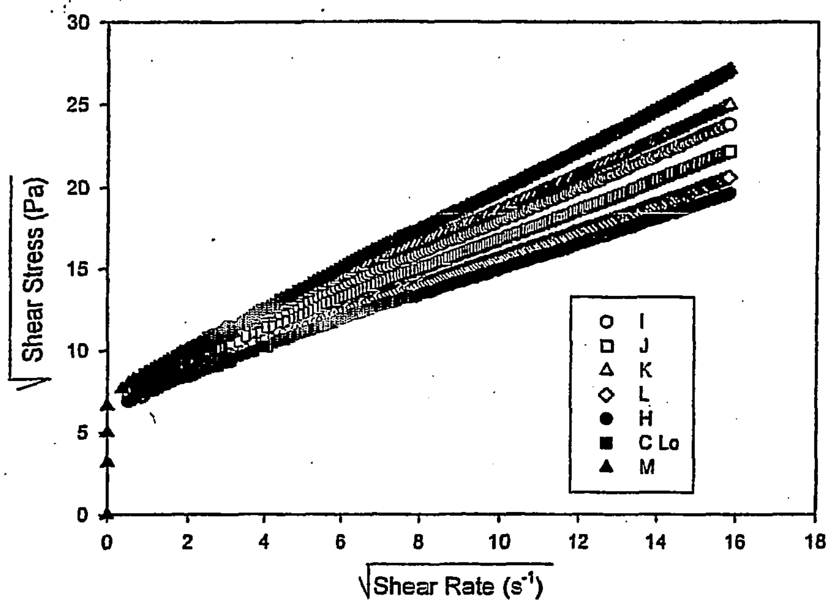


FIG. 7B

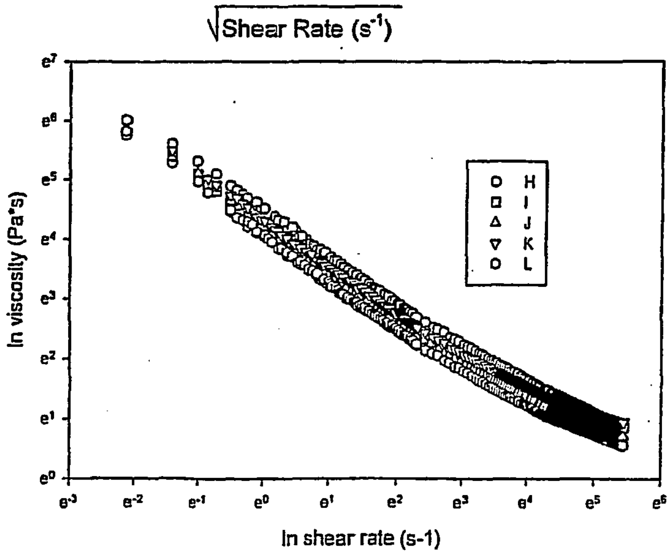

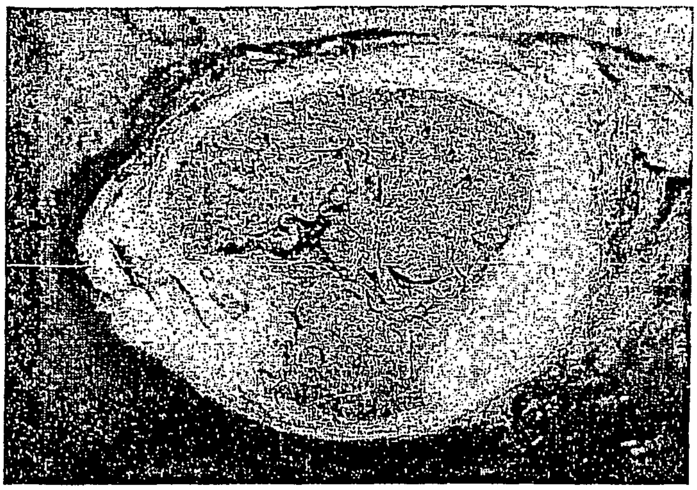

FIG. 8A

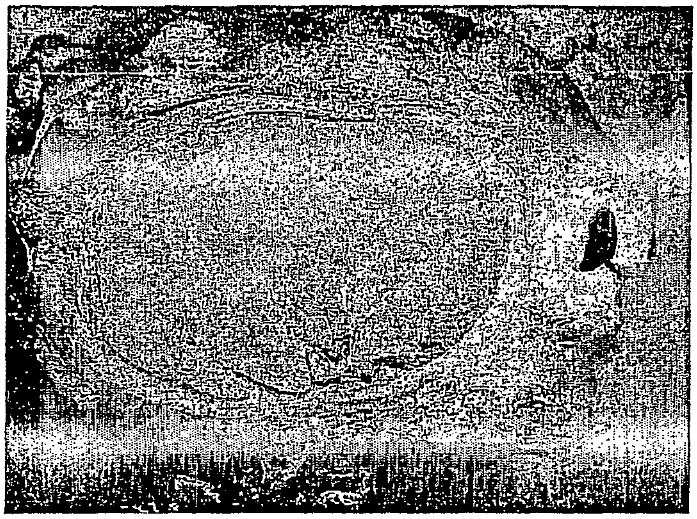

**FIG. 8B**

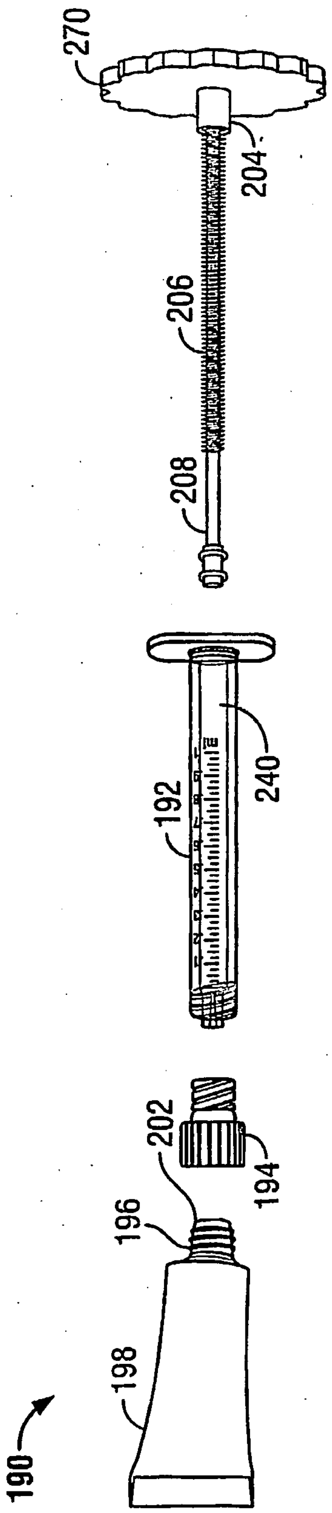
270
204
206
208
192
240
202
194
196
198
190

FIG. 9

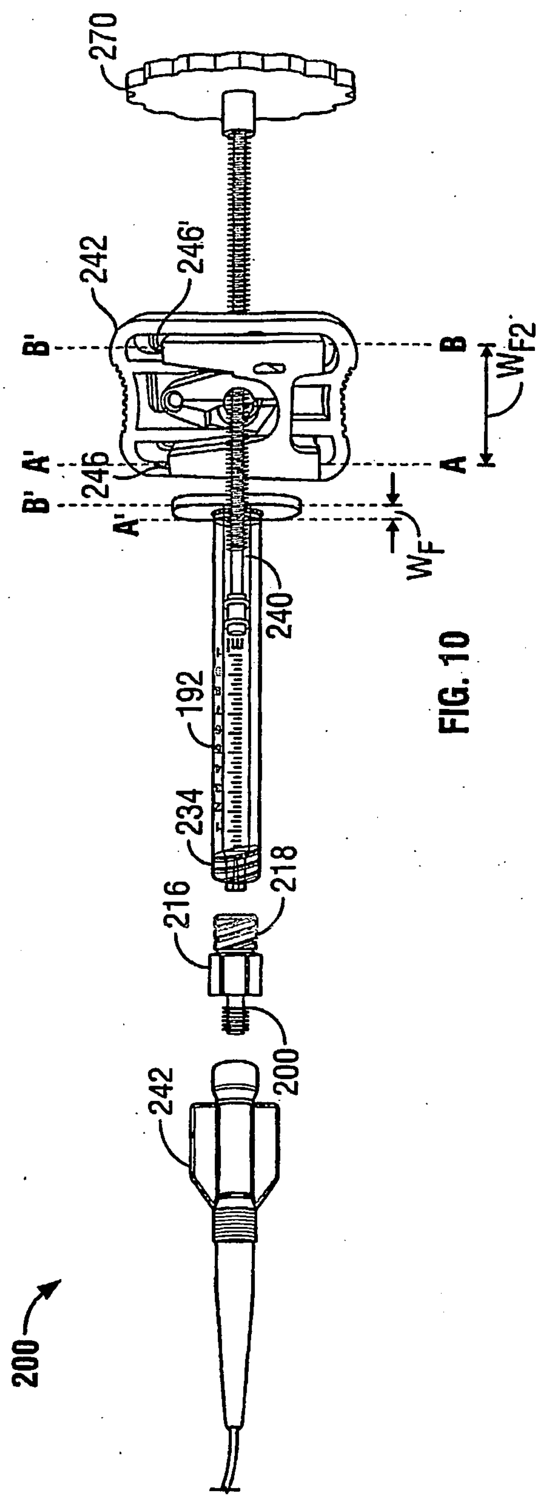
270
242
246'
B'
A'
246
B'
A'
B
A
$W_{F2}$
$W_F$
240
192
234
218
216
200
242
200

FIG. 10

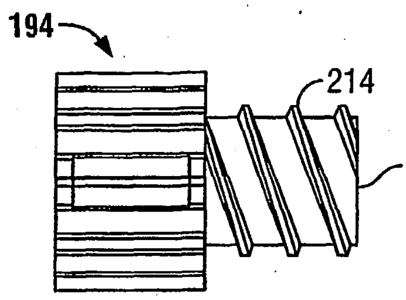


FIG. 11

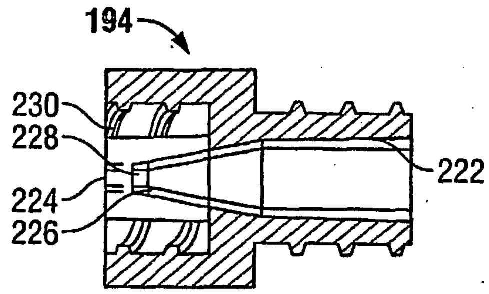


FIG. 12

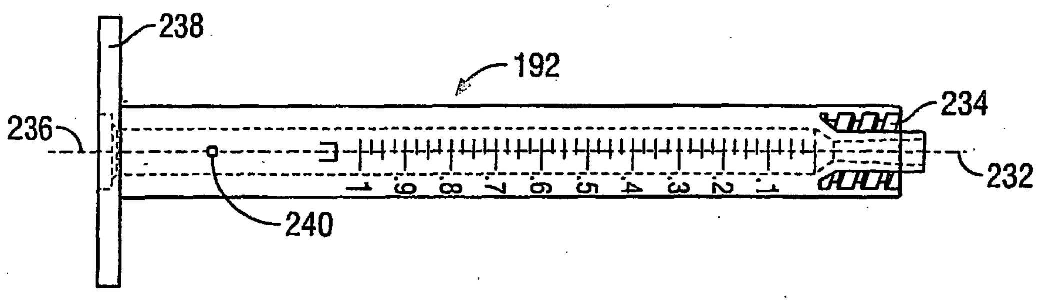


FIG. 13

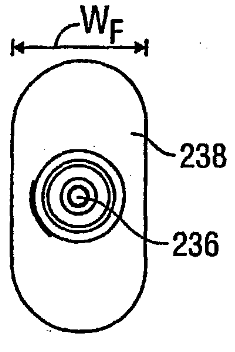


FIG. 14

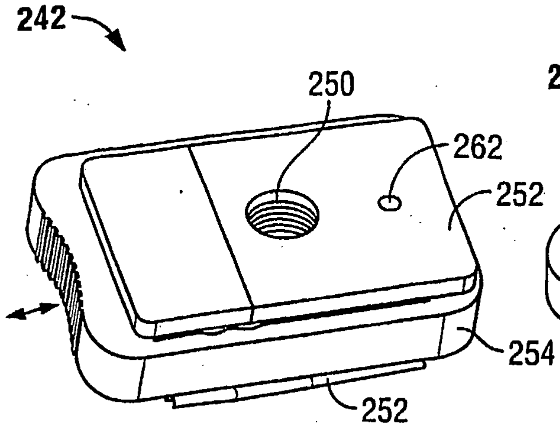


FIG. 15

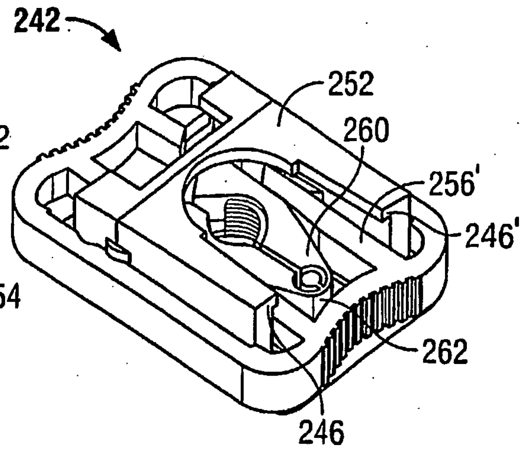


FIG. 16

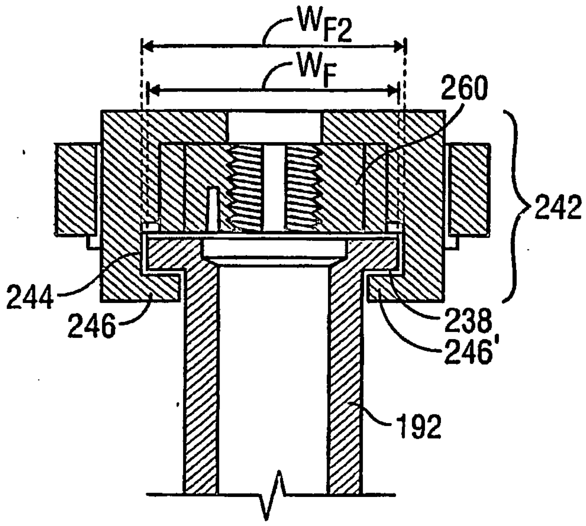


FIG. 17

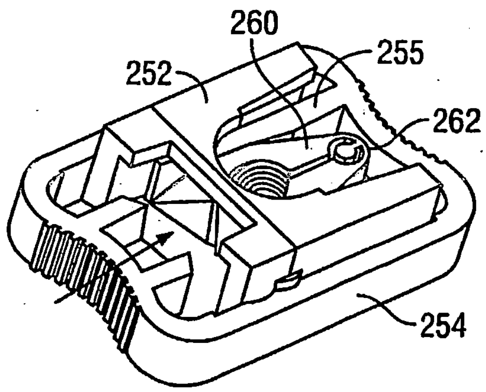


FIG. 18

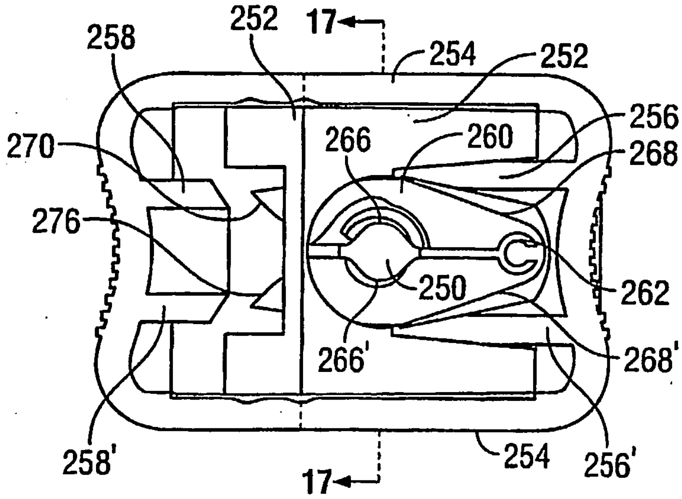


FIG. 19

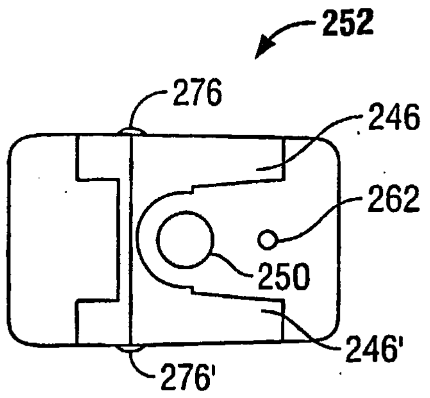


FIG. 20

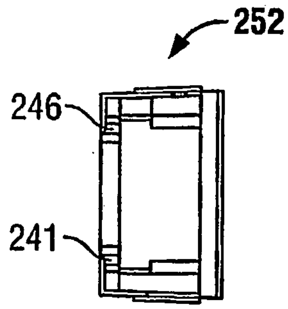


FIG. 21

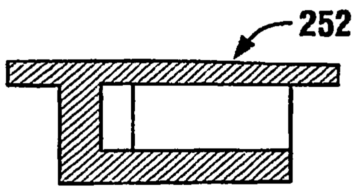


FIG. 22

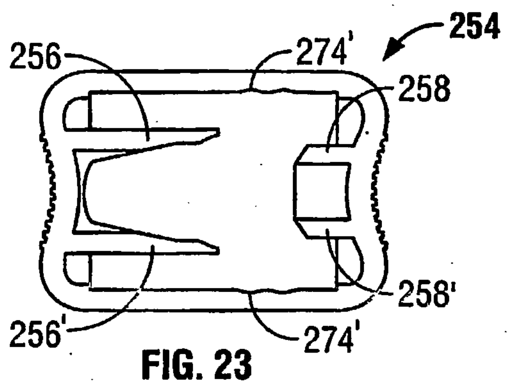


FIG. 23

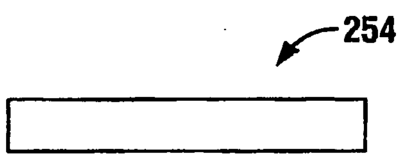


FIG. 24

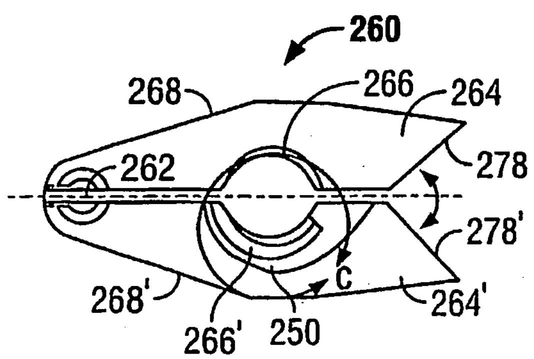


FIG. 25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60450289 B
- US 60461177 B
- US 60451310 B
- US 60461290 B
- US 60450626 B
- US 60461289 B
- US 60450288 B
- US 60451228 B
- US 60461288 B
- US 60461283 B
- US 5851508 A, Greff
- US 6645167 B, Whalen II
- US 6531111 B, Whalen, II
- US 20030039696 A, S.C. Porter
- US 68692903 A, C. Porter
- US 68754503 A, C. Porter
- US 5695480 A, Evans
- US 16265302 A, Greff
- US 6248800 B, Greff
- US 20020165582 A, Porter
- US 5667767 A, Greff
- US 5580568 A, Greff
- US 4938763 A, Dunn
- US 6231613 B
- US 6238335 B
- US 6595910 B
- US 6569417 B
- US 20030055386 A


### Non-patent literature cited in the description


- **HADEMMENOS ; MASSOUD.** The Physics of Cerebrovascular Diseases. Springer-Verlag, 1998
- **BIRD ; STEWART ; LIGHTFOOT.** Transport Phenomena. John Wiley & Sons, 1960
- **BRAUN ; ROSEN.** Rheology Modifiers Handbook: Practical Use and Application. William Andrew Publishing, 2000
- Cab-O-SIL Untreated Fumed Silica: Properties and Functions. *Cabot Corp,* 2000
- **MANDAI et al.** Direct Thrombosis of Aneurysms with Cellulose Acetate Polymer. *J. Neurosurg.,* 1992, vol. 77, 497-500
- **KINUGASA et al.** Direct Thrombosis of Aneurysms with Cellulose Acetate Polymer. *J. Neurosurg.,* 1992, vol. 77, 501-507
- **CASARETT ; DOULL'S et al.** Toxicology. Pergamon Press, 1975, 661-664
- **KINUGASA et al.** Early Treatment of Subarachnoid Hemorrhage After Preventing Rerupture of an Aneurysm. *J. Neurosurg.,* 1995, vol. 83, 34-41
- **KINUGASA et al.** Prophylactic Thrombosis to Prevent New Bleeding and to Delay Aneurysm Surgery. *Neurosurg,* 1995, vol. 36, 661
- **TAKI et al.** Selection and Combination of Various Endovascular Techniques in the Treatment of Giant Aneurysms. *J. Neurosurg.,* 1992, vol. 77, 37-42
- **CASSON.** Rheology of Dispersed Systems. Pergamon, 1959
- **BRAUN ; ROSEN.** Rheology Modifiers Handbook: practical use and application. William Andrew Publishing, 2000